Europäisches Patentamt

European Patent Office

Office européen des brevets

(11) Publication number: **0 256 115 B1**

## EUROPEAN PATENT SPECIFICATION

(45) Date of publication of patent specification: **19.12.90**

(51) Int. Cl.⁵: **A 61 B 5/08**

(21) Application number: **87901810.9**

(22) Date of filing: **21.01.87**

(86) International application number:
**PCT/US87/00217**

(87) International publication number:
**WO 87/04332 30.07.87 Gazette 87/17**

(54) **SINGLE POSITION NON-INVASIVE CALIBRATION TECHNIQUE FOR RESPIRATION MONITORING APPARATUS.**

(30) Priority: **21.01.86 US 820604**

(43) Date of publication of application:
**24.02.88 Bulletin 88/08**

(45) Publication of the grant of the patent:
**19.12.90 Bulletin 90/51**

(84) Designated Contracting States:
**AT BE CH DE FR GB IT LI LU NL SE**

(56) References cited:
**US-A-4 267 845**
**US-A-4 308 872**
**US-A-4 373 534**

(73) Proprietor: **NON-INVASIVE MONITORING SYSTEMS, INC.**
**1840 West Avenue**
**Miami Beach, FL 33139 (US)**

(72) Inventor: **WATSON, Herman**
**1842 West Avenue**
**Miami, FL 33139-1432 (US)**

(74) Representative: **Joly, Jean-Jacques et al**
**CABINET BEAU DE LOMENIE 55, rue d'Amsterdam**
**F-75008 Paris (FR)**

Courier Press, Leamington Spa, England.

# EP 0 256 115 B1

**Description**

Technical Field

This invention pertains to methods and apparatus for measuring respiration volume and, more particularly, to such methods and apparatus which measure respiration volume by separately measuring and then summing the contributions from a plurality of torso portions, such as the rib cage and abdomen. Most particularly, this invention pertains to a calibration technique for weighting signals indicative of the contributions from the torso portions whereby the sum of the signals is proportional to respiration volume.

Background Art

United States Patent No. 4,308,872 of January 5, 1982, entitled Method and Apparatus for Monitoring Respiration, a method is disclosed which comprises looping first and second extensible conductors about the rib cage and abdomen, separately and simultaneously measuring the inductances of the conductors during respiration, weighting the measured inductances to reflect the different contributions of the rib cage and abdomen to respiration volume, and summing the weighted measured inductances to obtain actual respiration volume.

As noted, practice of the technique disclosed in the patent requires weighting or calibrating the inductances measured by the abdomen and rib cage conductors. To effect calibration it is necessary to determine the weighting factors K and L to satisfy the following equation:

$$V = K \cdot RC + L \cdot AB$$

<div align="right">[EQUATION A]</div>

where V is total respiration volume, RC is the rib cage contribution to respiration volume as measured at the rib cage conductor and AB is the abdominal contribution as measured at the abdominal conductor. Patent No. 4,308,872 disclose a specific technique for determining the values for the weighting factors K and L.

In accordance with that disclosure, a spirometer is employed during the calibration procedure. With the patient in a first position, such as standing, a simultaneous set of readings are recorded from the outputs of the spirometer, the rib cage conductor, and the abdominal conductor. This is repeated with the patient in a second position such as supine. At this point, there are two sets of values for V, RC, and AB which satisfy Equation A. Thus, two equations having two unknowns, the constants K and L, may be written. From these, the weighting factors K and L may be determined by employing well known techniques for solving simultaneous equations. Thus:

$$K = \frac{AB_1 \cdot V_2 - AB_2 \cdot V_1}{RC_2\,AB_1 - RC_1\,AB_2} \qquad \text{[EQUATION B]}$$

$$L = \frac{RC_1 \cdot V_2 - V_1 \cdot RC_2}{AB_2\,RC_1 - AB_1\,RC_2} \qquad \text{[EQUATION C]}$$

The denominators of Equations b and d may, depending upon the recorded values, approach of equal zero. Clearly, when this happens, the values obtained for K and L will be inaccurate, thereby skewing any measurement based on such weighting factors. Thus, each time the denominations of Equations B and C approach or equal zero, a new set of readings must be taken, thereby increasing the time required for calibration.

U.S. Patent No. 4,373,534 of February 15, 1983, discloses an alternate method and apparatus for a graphing-based technique for determining the weighting factors K and L. As in the simultaneous equation technique of U.S. Patent No. 4,308,872, a spirometer or other device for independently measuring respiration volume is employed during the calibration procedure. With the subject in a first position, readings from the spirometer, the rib cage conductor and the abdominal conductor are simultaneously recorded for a plurality of breaths, preferably at least three in number. This is repeated with the subject in a second position. For each breath, the rib cage and abdominal readings are divided by the spirometer reading. That is, the values RC/V and AB/V are obtained for each breath, where V is the respiration volume as measured by the spirometer, RC is the rib cage reading from the uncalibrated rib cage conductor, and AB is the abdominal reading from the uncalibrated abdominal conductor. The points (RC/D, AB/V) for each breath are next plotted on a graph whose axes are RC/V and AB/V, and a line approximation is drawn through these points. The line may be drawn by visual approximation, although preferably it is determined by the least squares technique. The line is then extended through the x and y axes. The reciprocals of the x and y intercepts define the weighting factors K and L; i.e. the reciprocal of the intercept of the RC/V axis defines the weighting factor K for the rib cage and the reciprocal of the intercept of the AB/V axis defines the weighting factor L for the abdomen. Preferably, all of the foregoing calculations are carried out by a microprocessor or other data processor which performs the calculations and yields values for the weighting factors K and L.

<div align="center">2</div>

A drawback of the methods and apparatus disclosed in U.S. Patents Nos. 4,308,872 and 4,373,534 is the requirement that sets of data points or values — from the abdominal and rib cage conductors and from the spirometer or other respiration volume measurement device — be obtained with the subject for two different distributions of ventilation — i.e. in two separate positions. Where a subject's physiological condition prevents or dictates against movement from one position to another, however, calibration of the rib cage and abdominal conductor contributions cannot be readily carried out in accordance with the known methods.

Another drawback of these prior art methods and apparatus is that in certain applications, such as neo-natal monitoring, it is not practical to calibrate the apparatus using an independent respiration volume measuring device such as a spirometer. For example, since the above calibration techniques require airway connection to a spirometer or other similar device, significant time is required to carry out the procedure. This is often unacceptable to new-born nursery staff where time is at a premium.

Yet another problem with these calibration techniques and apparatus is that they rely on the assumption that all air movement in the respiratory system is between the rib cage and spirometer or the abdomen and the spirometer. In fact, there also exists movement of air between the rib cage and the abdomen during normal respiration. This RC—AB exchange of air is pendulluft that occurs continuously and with varying degree in respiration. The known methods do not incorporate an allowance for pendelluft.

Another known quantitative calibration technique is described in Single Position Tidal Breathing Calibration of the Respiratory Inductive Plethysmograph, Watson et al, Amer. Rev of Respiratory Diseases, Vol. 129, p. A256 (1984). According to that technique, with the subject in a single position or posture, readings from a spirometer (SP), the rib cage conductor (RC), and the abdominal conductor (AB) are simultaneously recorded for a predetermined period of preferably at least one full breath. The curves (SP, RC) and (SP, AB) are then plotted from the recorded data and each resulting curve is closed by a straight line connecting its beginning and end point. The resulting loop areas are then calculated as by integration. Then, using any selected data points simultaneously recorded from the spirometer (SP) and from the rib cage (RC) and abdominal (AB) conductors, weighting factors (for the rib cage scaling amplifier and/or for the abdomen scaling amplifier) may be determined. This calibration technique also suffers from the drawback that it requires a spirometer or other device measuring respiration volume at the mouth.

The object of the present invention is to provide an improved method for calibrating the signals from the rib cage and the abdomen without the drawbacks of the above-mentioned known techniques.

According to the invention, a method is provided of the type including means for providing a signal responsive to a rib cage dimension indicative of rib cage contribution to respiration volume, means for providing a signal responsive to an abdominal dimension indicative of abdominal contribution to respiration volume, and means for multiplying at least one of said rib cage and abdominal signals by a predetermined weighting factor for reflecting the relative contributions of said rib cage and abdomen to respiration volume, whereby an information representative of the respiration volume can be obtained from the sum of the weighted rib cage and abdominal signals, said method being characterized in that said weighting factors are determined by:

(a) totaling delta values for said rib cage signal and said abdominal signal over a baseline period of substantially steady state breathing, said delta values being parameters of said rib cage and abdominal signals indicative of the relative amplitude of those signals for each of a plurality of breaths over said baseline period; and

(b) dividing an average variability of the mean of the total of said delta values for one of said rib cage or abdominal signals by an average variability of the mean of the total of said delta values for the other of said rib cage or abdominal signals, and in that said other signal is multiplied by a weighting factor equaling the quotient derived from step (b).

Another object of the invention is to provide an apparatus capable of carrying out the above method. The apparatus is defined in claim 6.

Brief Description of the Drawings

In the drawings, wherein like reference numerals denote similar elements throughout the several views:

FIG. 1 is a diagrammatic representation of a portion of a system for non-invasively monitoring respiration volume;

FIG. 2 is a block diagram of a complete system for non-invasively monitoring respiration; and

FIG. 3 is a graphic representation illustrating the delta values for the rib cage and abdominal signals.

Best Mode For Carrying Out The Invention

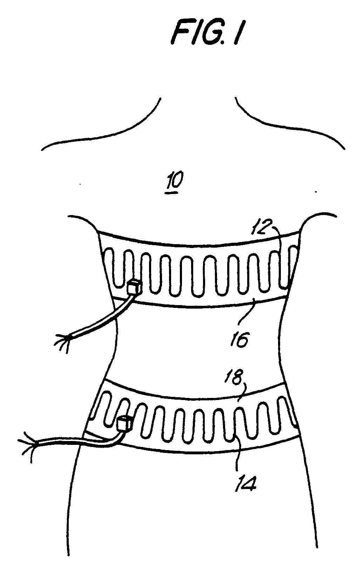

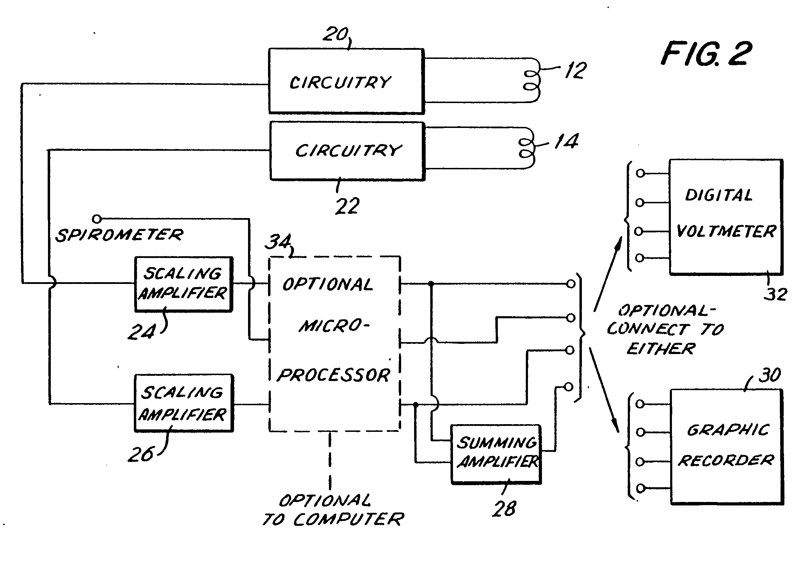

Referring now to the drawings, apparatus for measuring respiration volume of the type disclosed in U.S. Patent No. 4,308,872 is shown in FIGS. 1 and 2. Two extensible conductive loops 12, 14 are secured in any suitable fashion to elastic tubes 16, 18, respectively, such that the conductors 12 and 14 extend respectively about the rib cage and abdomen of the subject 10. As the subject 10 breathes, the elastic tubes 16, 18 and conductive loops 12, 14 expand and contract, resulting in changes in the inductances of the loops. After the inductance of each loop is converted to a proportional signal, the signals are calibrated and then summed to provide a signal indicative of tidal volume. Calibration of the signals from the rib cage and abdomen conductors 12 and 14, respectively, is necessary because the relative contributions of the rib cage and abdomen to tidal volume vary from subject to subject and even in a single subject with different postures, e.g. standing, supine, etc.

Suitable apparatus for converting the inductances of the conductors 12 and 14 to proportional electrical signals, calibrating those signals to reflect the proper relative contributions, and then summing those signals to provide a signal indicative of tidal volume are known to those of ordinary skill in the art. One such apparatus is disclosed in U.S. patent no. 4,308,872. Another suitable apparatus is marketed by Nims, Inc., Miami Beach, Florida under the model designation Respirograph™. Such apparatus are generically illustrated in block diagram form in FIG. 2, where the blocks 20, 22 represent, respectively, appropriate circuitry for converting the inductances of the rib cage and abdominal conductors 12, 14 to proportional electrical signals suitable for further processing. The scaling amplifiers 24, 26 represent circuitry for calibrating the signals from the rib cage and abdomen, respectively, to reflect the relative contributions of the rib cage and abdomen to tidal volume. Once the scaling amplifiers 24, 26 are properly calibrated, the resulting signals may be summed, as by the summing amplifier 28 in FIG. 2, to yield a signal indicative of tidal volume. The output signal from the summing amplifier 28 as well as the output signals from the two scaling amplifiers 24 and 26 may then be displayed as on a graphic recorder 30 or a digital voltmeter 32. Optionally, a microprocessor 34 may be incorporated in the apparatus for summing the signals from the scaling amplifiers 24, 26 and/or further processing those signals for diagnostic purposes, all in accordance with techniques known to those of ordinary skill in the art. If the microprocessor 34 is used to sum the signals from the scaling amplifiers, the summing amplifier 28 may be eliminated.

Turning to the calibration technique of the invention, from Equation A it is known that:

$$V = (K \times RC) + (L \times AB)$$

where V is total respiration volume or tidal volume, RC is the rib cage contribution to respiration volume as measured by the rib cage conductor and AB is the abdominal contribution to respiration volume as measured by the abdominal conductor. K and L are calibration factors for the rib cage and abdomen, respectively. Another way of expressing this relationship is:

$$V = Mx[(ZxRC) + AB] \qquad \text{[EQUATION D]}$$

where MxZ is equal to K and M is equal to L. It will be apparent that Equation D separates the calibration components into a proportionality factor Z and a scaling factor M. Using this approach, calibration may be viewed as a two step process. The first step is the determination of the correct proportionality factor Z satisfying Equation D, such that

$$V \sim (ZxRC) + AB \qquad \text{[EQUATION E]}$$

In other words, the proportionality factor Z defines the correct relative contributions of the rib cage (RC) and abdomen (AB) to tidal volume (V). Classically, determination of the proportionality factor Z is determined by an isovolume calibration technique in which the subject breathes against a closed airway i.e. with no volume movement at the mouth, whereby V=0. In other words, during an isovolume manouver, the only movement of volume is between the rib cage and abdomen compartments, i.e. pendelluft, since no air escapes through the mouth. Under these conditions, Equation D becomes:

$$O = (ZxRC) + AB \qquad \text{[EQUATION F]}$$

$$Z = -AB/RC \qquad \text{[EQUATION G]}$$

So, by recording the readings from the rib cage and abdomen conductors during the isovolume manouver, the proportionality factor Z can be determined from Equation G. Once Z is determined, the quantity (ZxRC)+AB can be calculated for any point in time from the recorded values of the rib cage and abdomen conductors. Since we know from Equation E that (ZxRC)+AB is always proportional to tidal volume V, a determination of that quantity provides a valuable diagnostic tool. For example, as those of ordinary skill in the art will appreciate, from this quantity obstructive and central apneas can be diagnosed, RC as a percent of tidal volume V can be calculated, and increases and decreases in relative tidal volume V can be assessed.

The difficulty with this approach is that an isovolume manouver requires breathing against a closed airway, which is not always practical, as in neo-natal and critical care applications.

If, on the other hand, the proportionality factor Z and hence the quantity (ZxRC)+AB is to be determined without an isovolume manuver, it would appear from Equation E that a measurement of tidal volume must be taken, otherwise there will be a single equation with two unknowns, namely, proportionality factor Z and tidal volume V.

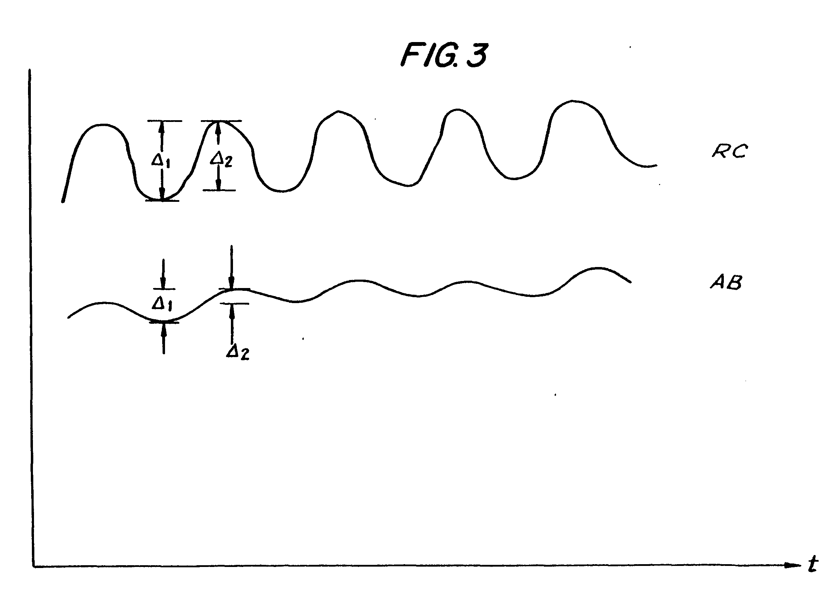

In accordance with the present invention, this problem is solved as follows. The rib cage (RC) and abdomen (AB) signals are recorded for a large number of breaths during an initial baseline period. For example, 250 breaths may be measured during quiet breathing over a 10 minutes interval. Actually, breathing during the baseline period need not be quiet, as long as it is steady state. For example, the baseline could be derived from breaths recorded during 10 minutes of exercise. During this baseline period, the uncalibrated signals from the rib cage (RC) and abdomen (AB) conductors are recorded. Referring to FIG. 3, for each of these signals, two values or breath "deltas" are calculated for each breath, ($\Delta_1$) being the difference between the signal at the beginning and end of inspiration, the other ($\Delta_2$) being the difference between the beginning and end of expiration. These delta values are then totaled separately for each signal. Assuming 250 breaths during baseline, and since there are two delta values for each breath, the total for each signal will be computed by adding 500 delta values. Where the delta values described above are preferred, it should be appreciated that the delta values for the rib cage and abdomen is here intended to mean any parameter of the uncalibrated rib cage and abdomen signals indicative of the relative amplitude of those signals for each of a plurality of breaths taken over a baseline period.

If actual tidal volume were also recorded during baseline, as by spirometry, and the deltas for tidal volume totaled and the mean determined, the following relationship would apply:

$$\text{Mean SP} = \text{Mean RC} + \text{Mean AB} \qquad \text{[EQUATION H]}$$

Where SP is actual tidal volume as determined, e.g. by spirometry. Since the mean values in Equation H are derived from uncalibrated signals, calibration factors are required if the lefthand side in Equation H is to equal the righthand side.

The standard deviations (SD) of the mean values for the tidal volume (SP) rib cage (RC) and abdomen (AB) signals can be calculated. Herein, these will be expressed, respectively, as SD (SP), SD (RC), and SD (AB). If tidal volume (SP) is constant for all breaths recorded during baseline, Equation H can still be computed and the standard deviation of tidal volume, SD (V), is 0.

This situation is analogous to Equation F which, as explained above, applies to the isovolume manouver where V=0. In particular, by considering the standard deviation of a constant tidal volume SD (V), which is also 0, the pendulluft occurring during normal breathing creates a situation analogous to the isovolume situation of Equation F, and taking the variance (Var) of both sides, yields:

$$\text{Var (ZxRC)} = \text{Var (AB)} \qquad \text{[EQUATION J]}$$

Where variance (Var) is equal to (SD)$^2$. Equation J may be expressed as

$$Z^2 x \text{Var (RC)} = \text{Var (AB)} \qquad \text{[EQUATION K]}$$

By taking the square root of both sides

$$Z x \text{SD (RC)} = \text{SD (AB)} \qquad \text{[EQUATION L]}$$

Which yields

$$Z = \text{SD (AB)}/\text{SD(RC)} \qquad \text{[EQUATION M]}$$

It should be appreciated that the standard deviations of the means of the rib cage and abdomen signals is indicative of the average variability of those signals. Accordingly, any analysis that provides an indication of average variability of those signals may be used in lieu of computing standard deviations. Put into other words, it will be apparent from Equation M that the proportionality constant Z for solution of Equation E can be calculated, assuming constant tidal volume breathing during baseline, from the ratio of the standard deviations of AB and RC in a manner analogous to the isovolume manuver, but without the requirement that the airway be blocked.

Of course, taking spirometry readings with a spirometer during baseline defeats an objective of the calibration technique in accordance with the invention, namely, to calibrate without needing a spirometer.

However, baseline constant tidal volume assumption required to practice the calibration technique of the invention without recording actual tidal volumes by means of a spirometer can be satisfied by removing wild points from the 500 delta values of AB and RC computed during baseline as by eliminating values outside of 1.5 standard deviations of the uncalibrated sum of the RC and AB components. With those delta values excluded, the remaining delta values for RC and AB are separately totaled, the means determined, and the standard deviations for the means calculated. The proportionality factor Z can then be calculated from Equation M.

Once the proportionality factor Z is known, the quantity (ZxRC)+AB will always be proportional to actual tidal volume. See Equation E. This quantity can be continuously monitored on a real time basis from the real time rib cage and abdomen signals generated by the apparatus of FIG. 2. Preferably, this quantity is expressed as a percent of (ZxMean RC)+Mean AB where Mean RC and Mean AB are, respectively, the means of the totals of the uncalibrated RC and AB delta values generated at baseline, but excluding the wild points. This is sufficient for the bulk of diagnostic work, such as detecting obstructive and central apneas, hypoapeneas, and variations in tidal volume, the latter being important as a diagnostic tool in a wide variety of disorders.

Referring to FIG. 2, assuming the scaling amplifiers have initially been set to unity gain, once the proportionality factor Z is determined, the scaling amplifier 24 for the rib cage is adjusted to Z. From Equation E, we then know that the sum of the signals from the scaling amplifiers at the output of the summing amplifier 28 will be proportional to tidal volume. This completes the calibration procedure.

While for most purposes a quantitative determination of tidal volume is not necessary, quantitative calibration can be achieved once the proportionality factor Z is known. In particular, referred to Equation D, by taking a single measurement of actual tidal volume V, the scaling factor M can be calculated, since ZxRC and AB are available, respectively, at the output of the rib cage and abdomen scaling amplifiers. In other words, there is then only a single unknown in Equation D, the scaling factor M, which is calculated as:

$$M = V/[(ZxRC)+AB]. \qquad \text{[EQUATION N]}$$

One simple way to take a measurement of actual tidal volume is to simply have the subject inhale a known quantity of air, as from a syringe. Before the subject inhales, this quantity is input to the apparatus of FIG. 2 at the "spirometer", input. The microprocessor 34 can then perform the calculation of Equation N from the values of Z, RC and AB at the end of inspiration from the syringe.

The scaling factor M may be set in the FIG. 2 apparatus by multiplying the gain of the summing amplifier 28 by scaling factor M, whereupon the output of the summing amplifier will be a semiquantitative indication of tidal volume. The term semi-quantative is used because it has been determined that tidal volume computed in this fashion is ± 10% of actual tidal volume as determined by spirometry.

Desirably, the accuracy of the proportionality factor Z is continuously monitored by repeatedly recalculating the proportionality factor at five minute intervals and printing out the resulting value. After the initial calibration procedure, the recalculated value for the proportionality factor Z during each subsequent five minute interval should be 1.0. In the event of a change in the position of the patient or other condition that varies the proportionality factor Z, that will exhibit itself as a reading of Z above or below 1.0. If the variations are too large, i.e. if Z is less than about .7 or greater than about 1.3, the calibration routine can be rerun. Suitable visual or audio alarms can be incorporated in the apparatus of FIG. 2 to indicate such excessive variations.

As noted above, the calibration technique in accordance with the present invention is currently utilized in connection with an apparatus for monitoring respiration sold by Nims, Inc., Miami Beach, Florida, under their model designation Respigraph™. The Respigraph incorporates a microprocessor. Preferably, the microprocessor is programmed to carry out the calibration technique in accordance with the present invention. A program listing for a suitable program for carrying out the calibration function appears below in Table A. The program is in assembly language for a Z80 microprocessor as manufactured by Zylog, Inc.

FILE: QDC.ASM

```
    ;
    ;
    ;
    .IDENT          QDC
    .INSERT         SP80.ASM
    .INSERT         FPMAC.SRC
    .INSERT         CALCOM.ASM
    .INSERT         CKCOM.ASM
    .INSERT         VTCCOM
    .INSERT         SELCOM.ASM
    .INSERT         VARCOM.COO
    .INSERT         OLQCOM
    .INSERT         SYCQM
    .INSERT         PKCOM
    ;
    .INSERT         EFFCOM
    .INSERT         SIOCOM
    .INSERT         HRDCOM
    .EXTERN         VALCOL, VALFND, TVON, SAVECL, LPON, LPOFF, TVOFF
    .EXTERN         FIND
    .EXTERN         DERIV5, PRTIME
    .EXTERN         SAVECL
    ;
    .EXTERN         VAL
    ;
    ;
    ;
    .ENTRY          QDC1, QDC2, LQDC1
    .ENTRY          BASE
    ;
    .EXTERN         RTON, RTOF, RTICK
    ;
    .EXTERN         QDCAL, SYST, ANSOUT
    ;
    ;
    ;
    .EXTERN         SRTINIT, SRTPLOT, EXEC
    .EXTERN         LABEL
    .EXTERN         TDHE
    .EXTERN         PTRUPD, GETP2, ROUND
    ;
    ;
    FINIT
    ;
    ;
    ;
QDC:
    ;
    ;THESE JUMPS MUST BE THE 1ST THINGS:
    JMP             QDC1
    JMP             QDC2
    JMP             VAL
    JMP             LQDC1
    JMP             ISOV1
    JMP             BASE
```

```
;
;
;
;
ISOV1:
    XRA         A
    STA         BSEFLG
    MVI         A, 0FFH
    STA         ISOFLG
    MVI         A, 0
    STA         WTFLG
    JMP         QDCCOL
;
;
;
;
BASE:
    MVI         A, 0FFH
    STA         BSEFLG
    MVI         A, 0FFH
    STA         WTFLG
    XRA         A
    STA         ISOFLG
    JMP         QDC3COL

;
;
;
;
QDC1:
    XRA         A
    STA         BSEFLG
    MVI         A, 0
    STA         WTFLG
    STA         ISOFLG
    JMP         QDCCOL
;
LQDC1:
    XRA         A
    STA         BSEFLG
    MVI         A, 0FFH
    STA         WTFLG
    XRA         A
    STA         ISOFLG
    JMP         QDCCOL
;
QDCCOL:
;
    XRA         A
    CMA
    STA         QUALFLG     ;QUALITATIVE
    XRA         A
    STA         BSEFLG
;
```

```
;
QDC3COL:
;
;
;
;START THE DATA COLLECTION
;
;

    XRA        A
    STA        VALFLG
    STA        POS1       ;NOT IN SINGLE POSITION
    LXI        H, 0
    SHLD       NBERS       ;# VALUES IN XVALUES & YVALUES
    ;
    LXI        H, 0
    SHLD       TIME
    LXI        H, 0
    SHLD       MAXCNT
    ;
    LDA .      BSEFLG
    IF ( .A, IS, ZERO)      ;NOT COLLECTING BASELINE
;INIT THE TRANSDUCESERS
    MVI        A, 10
    CALL       SYST
;THIS RESETS CALS TO 1 AND 1 SO AFTER THIS IS EXECUTED, ALL OTHER
;  CALIBRATIONS ARE NULLIFIED
    XRA        A
    STA        CALTYPE
    STA        LASTCAL      ;RIGHT NOW UNCALBRATED
    STA        GOODBASE        ;NO LONGER HAS GOOD BASELINE
    ;
    CALL       LPON
    FPRN       RES, RESPT
    CALL       LPOFF
    ENDIF
    ;
    CALL       SETUP
    CALL       SRTINIT      ;INIT RT. PLOT
    LOOP
    CALL       SRTPLOT
    ;
    LDA        BFLG
    IF ( .A, IS, NZERO)
;UPDATE # DELTAS AND ELAPSED TIME
    MVI        A, 3
    STA        OPCODE
    CALL       ANSOUT
    ELSE
    LDA        SECFLG
    IF ( .A, IS, NZERO)
    MVI        A, 3
    STA        OPCODE
```

9

```
        CALL            ANSOUT
        LDA             FLSHFLG
        DCR             A
        STA             FLSHFLG
        IF (.A, IS, ZERO)
                LXI     H, TOPLFT+53
                LDA     BSEFLG
                IF ( .A, IS, ZERO)
                  LDA   ISOFLG
                  IF ( .A, IS, ZERO)
                    LXI D, CLBMESS
                  ELSE
                    LXI D, ISOMESS
                  ENDIF
                ELSE
                    LXI D, BSEMESS
                ENDIF
                CALL    LABEL
                MVI     A, 2
                STA     FLSHFLG
        ELSE
                LXI     H, TOPLFT+53
                LXI     D, BLANKS
                CALL    LABEL
        ENDIF

    ENDIF
    ENDIF
;
    LDA             GFLG        ;= 0FFH THEN CLOCK
                                ;STILL ON
    EXITIF( .A, IS, ZERO)

    ENDLOOP
;
;
;NOW DATA COLLECTION PHASE HAS BEEN COMPLETED
;MAXCNT HOLDS THE NUMBER OF MAX POINTS COLLECTED
;
;IRSRV USES CHANNEL SPBUFF FOR DETECTIONS
;SPMAX HOLDS THE DELTAS FROM SPBUFF
;RCMAX HOLDS THE DELTAS FROM RCBUFF
;ABMAX HOLDS THE DELTAS FROM ABBUFF
;
;CLEAR THE SCREEN
    CALL            TVON
    CALL            TVOFF

;
    LDA             BSEFLG
    IF ( .A, IS, ZERO)
    LDA             ISOFLG
    IF ( .A, IS, NZERO)        ;THIS WAS AN ISOVOLUME MANUEVER
      JMP           ISOCALC
```

10

```
        ENDIF
        ENDIF
    ;
        LHLD            MAXCNT
        LXI             D, 3
        ORA             A
        DSBC            D
        IF (PSW, IS, CARRY)
    ;
            LDA             WTFLG
            IF ( .A, IS, ZERO)
                MVI             A, 1
                STA             OPCODE      ;NOT ENOUGH DELTAS
                CALL            ANSOUT
                MVI             A, 0
                STA             OPCODE      ;PRINT MENU ON SCREEN
                CALL            ANSOUT
            ENDIF
            STC
            RET
        ENDIF
    ;
    ;
    ;
    ;THERE ARE 2*(MAXCNT -1) FLOATIND PT VALUES IN XVALUES
    ;AND YVALUES FOR THIS RUN
        LHLD            MAXCNT
        SHLD            NBERS       ;TOTAL # STORED
        CALL            QDCAL
    ;RETURN WITH CARRY SET IF BAD FOR ANY REASON
    ;
    ;
    ;ORA             A
    ;MVI             A, 0
    ;STA             OPCODE      ;PRINT MENU ON SCREEN
    ;CALL            ANSOUT
                    RET
    ;
    ;
    ;
    ;
    ISOCALC:
        LDA             WRAPF
        IF ( .A, IS, NZERO)     ;THEN 400 PTS AND RIPT PTS TO 1ST
                                ;DATA PT
            LXI             H, 400
            SHLD            CNT
            LHLD            RIPT
            SHLD            ROPT
        ELSE
    ;TIME HOLDS # OF DATA POINTS AND 1ST PT AT OFFSET = 0
            LHLD            TIME
            SHLD            CNT
```

```
        LXI             H, 0
        SHLD            ROPT
        ENDIF
;
        FFOA            0
        FSTA            XSUM
        FSTA            YSUM
        FSTA            XXSUM
        FSTA            XYSUM
        FSTA            XCNT
LOOP1:
        CALL            GETX
        CALL            GETY
        CALL            XYPAIR
;
        LHLD            ROPT
        LXI             B, RSIZE
        CALL            PTRUPD
        SHLD            ROPT
        DSKZ            CNT, LOOP1
;
        CALL            LSTFIT
        FLDA            SLOPE
        FABS
        FSTA            ABCAL
        FFOA            1
        FSTA            RCCAL
;PRINT IT ON SCREEN
        MVI             A, 4
        STA             OPCODE
        CALL            ANSOUT
;
        CALL            SAVECL
        IF (PSW, IS, NCARRY)      ;THEN GOOD VALUES
        MVI             A, 3        ;ISOV QUAL
        STA             CALTYPE
        MVI             A, 0FFH
        STA             LASTCAL       ;GOOD CALIBRATION
        XRA             A
        CMA
        STA             QUALFLG
        CALL            LPON
        CALL            PRTIME
        FPRN            ISV, ISVPT
        CALL            LPOFF
        ORA             A
        ENDIF
;
        RET
```

```
;
;
;
;
ISV:            .ASCIS '
                QUALITATIVE ISOVOLUME CALIBRATION
                RCCAL = ∂D3
                ABCAL = ∂D3
            '
ISVPT: .WORD    RCCAL, ABCAL
;
;
GETX:
    LXI         H, ABBUFF
    LDED        ROPT
    DAD         D
    MOV         E, M
    INX         H
    MOV         D, M
    SDED        XVAL
    FILA        XVAL
    FLOT
    FSTA        XVAL
    RET
;
GETY:
    LXI         H, RCBUFF
    LDED        ROPT
    DAD         D
    MOV         E, M
    INX         H
    MOV         D, M
    SDED        YVAL
    FILA        YVAL
    FLOT
    FSTA        YVAL
    RET
;
XYPAIR:
    FLDA        XVAL
    FLDB        XSUM
    FADD
    FSTA        XSUM
;
    FLDB        YVAL
    FLDA        YSUM
    FADD
    FSTA        YSUM
;
    FLDB        XVAL
    FLDA        XVAL
    FMUL
    FLDB        XXSUM
    FADD
    FSTA        XXSUM
;
```

13

```
        FLDB            XVAL
        FLDA            YVAL
        FMUL
        FLDB            XYSUM
        FADD
        FSTA            XYSUM
    ;
        FLDB            XCNT
        FFOA            1
        FADD
        FSTA            XCNT
        RET

    LSTFIT:
        FLDA            XSUM
        FLDB            XSUM
        FMUL
        FPSH
    ;
        FLDA            XCNT
        FLDB            XXSUM
        FMUL
        FSWP
        FPOP
    ;
        FSUB
        FPSH
    ;
        FLDA            XSUM
        FLDB            YSUM
        FMUL
        FPSH
    ;
        FLDA            XCNT
        FLDB            XYSUM
        FMUL
        FSWP
        FPOP
    ;
        FSUB
        FSWP
        FPOP
        FDIV
        FSTA            SLOPE
        RET

    ;
    QDC2:

    ;THIS CAN BE DONE AFTER EITHER A QDC1 (CALTYPE = 1/2)
    ;   OR AFTER AN ISOVOLUME (CALTYPE = 3/4)
    ;
    ;IF CALTYPE = 0, THEN HAVEN'T CALIBRATED THE RC TO AB
```

```
        LDA             CALTYPE
        IF ( .A, IS, ZERO)
            MVI         A, 2
            STA         OPCODE
            CALL        ANSOUT
            RET
        ENDIF
        ;
        ;NOW COLLECT DATA AS IF VALIDATING
        CALL            VALCOL
        ;
        LHLD            MAXCNT
        LXI             D, 2
        ORA             A
        DSBC            D
        IF (PSW, IS, CARRY)         ;THEN LESS THAN 2 BREATHS
            MVI         A, 1
            STA         OPCODE
            CALL        ANSOUT
            MVI         A, 0
            STA         OPCODE          ;PRINT MENU ON SCREEN
            CALL        ANSOUT
            RET
        ENDIF
        ;
        ;NOW FIND THE RATIOS OF SUM/KNOWN VOLUME
        CALL            VALFND
        ;RETURNS WITH AREG=# OF VALUES USED IN SUMMATION
        ;
        FSTA            FCNT
        ;
        ;.
        ;NOW XVALUES HOLDS ALL OF THE RATIOS, XSUM HOLDS SUM OF ALL XVALUES
        ;XXSUM HOLS SUM OF SQUARES OF ALL XVAUES, AND FCNT HOLS THE NUMBER
        ;OF VALUES USED IN THE SUMMATION
        ;
        ;COMPUTE THE MEAN RATIO
        FLDB            XSUM
        FLDA            FCNT
        FDIV
        FSTA            MEAN
        ;;NOW KNOW THAT SUM NEEDS TO BE MULTIPLIED BY 1/MEAN, SO
        ;RCCAL = CURRENT RC GAIN (RCGNF) * 1/MEAN
        ;ABCAL = CURRENT AB GAIN (ABGNF) * 1/MEAN
        FFOA            1
        FATB
        FLDA            MEAN
        FDIV
        FLDB            RCGNF
        FMUL
        FSTA            RCCAL
        ;
        FFOA            1
```

```
    FATB
    FLDA            MEAN
    FDIV
    FLDB            ABGNF
    FMUL
    FSTA            ABCAL
;
    MVI             A, 4
    STA             OPCODE
    CALL            ANSOUT
;
    CALL            SAVECL
    IF (PSW, IS, NCARRY)        ;THEN GOOD
    CALL            LPON
    CALL            PRTIME
    FPRN            VL2, VL2PT
    CALL            LPOFF
    LXI             H, 0
    SHLD            NBERS
    XRA             A
    STA             QUALFLG         ;NOT QUALITATIVE, IT IS QUANTITATIVE CAL
;
;
;
    LDA             CALTYPE
    IF ( .A, LT, 3)              ;THEN QDC METHOD
    MVI             A, 2        ;NOW ITS QDC QUANT
    STA             CALTYPE
    MVI             A, 0FFH
    STA             LASTCAL         ;GOOD CAL
    ELSE                            ;ISOVOLUME METHOD
    MVI             A, 4
    STA             CALTYPE         ;NOT ITS ISOV QUANT
    MVI             A, 0FFH
    STA             LASTCAL         ;GOOD CAL
    ENDIF
;
;
;
;MULT THE CURRENT VALUE OF VTLIMIT BY 1/MEAN SO WILL BE SCALED OK
;   NOTE: VTLIMIT IS INITIALLY SET WHEN DOING QDC1
;
;THIS IS FOR INITIALLIZING THE HYPOPNEA VOLUME FOR THE 1ST 5 MINUTES;
    FLDB            VTLIMIT
    FLDA            MEAN
    FDIV
    FSTA            VTLIMIT
;***************************************************************
;PROBLEM: WHAT IF DID ISOVOLUME BEFORE HERE, THEN VTLIMIT WAS NOT
            EVER INITIALIZED ????
;***************************************************************
    ENDIF
;
```

```
        ;MVI            A, 0
        ;STA            OPCODE      ;PRINT MENU ON SCREEN
        ;CALL           ANSOUT
                        RET
        ;;
        ;
        VL2:            .ASCIS '
          QUANTITATIVE CAL FACTORS
          RC CAL = ∂D3
          AB CAL = ∂D3
        ,

        VL2PT:
          .WORD         RCCAL, ABCAL
        ;
        ;
        ;NOTE:          MCA VALUES OF RCINVOL, ABINVOL, AND SMVOL WILL
                        BE STORED BEGINNING AT ADDR = XVALUES
        ;               XVALUES + YVALUES = 1600 BYTES
        ;               I WILL STORE UP TO 1500 BYTES
        ;
        ;               == > 500 DELTAS = 250 INSP DELTAS * 6 BYTES = 1500 BYTES
        ;               MCAADDR = ADDRESS OF NEXT TO STORE
        ;
        ;
        ;
        ;
        ;
        SETUP:
        ;
        ;THIS IS ONLY USED WHEN CALIBRATING QDC METHOD
        ;
        ;SETUP THE CLOCK
        ;
        ;SAMPLE AT 20 PTS PER SEC
          PUSH          H
        ; INIT REAL-TIME BUFFER POINTERS
        ; MODIFIED 09-24-81
        ;FOR PEAK FLOWS:
          LXI           H, DLTIMES
          SHLD          TMPTR
          LXI           H, 0
          SHLD          TTPOS
          SHLD          TTNEG
          SHLD          TEMPK
          SHLD          TTPTM
          SHLD          TTNTM
          SHLD          PKTIME
        ;
          LXI           H, 0
          SHLD          DLTIPTR     ;OFFSET TO STORE NEXT DELTA
          XRA           A
          STA           DLTFULL     ;DELTA RING BUFFER NOT FULL YET
```

```
        CMA
        STA         FSTMN
;
        LXI         H, XVALUES      ;WILL USE THIS BUFFER TO
        SHLD        MCAADDR         ;MCA VALUES
;
        LXI         H, 0
        SHLD        SPTEMP
;
        XRA         A
        STA         FRSTMX          ;1ST MAX NOT FOUND
;
        LXI         H, TMBUFF
        MVI         M, 0
        INX         H
        MVI         M, 0

;
        MVI         A, 2
        STA         FLSHFLG
;
;
        XRA         A
        STA         WRAPF
        STA         SECFLG
        CMA
        STA         BFLG
        LXI         H, 0
        SHLD        ELPTM
        SHLD        ELPMIN
        SHLD        SECONDS
;
        LXI         H, 0
        SHLD        RIPT
        SHLD        ROPT
        SHLD        ODRIPT
        LXI         H, 0
        9HLD        TIME
;INIT VARIABLES FOR PICKING MAX AND MINS
        XRA         A
        STA         MAXB
        STA         PTF         ;START BY LOOKING FOR MAX PT
        STA         SLOPEF      ;INIT MAX NOT FOUND
;
        XRA         A
        STA         INTFLG
        CMA
        STA         GFLG        ;INDICATES CLOCK ON
;
        LXI         H, IRSRV
        CALL        RTON        ;TURN ON CLOCK
;
        POP         H
```

```
        RET
        ;
        ;
        ;
CLKSTP:
        ;STOP DATA COLLECTION
        ;TURN CLOCK OFF
        ;
        ;MODIFIED 09-24-81
        ;
        CALL        RTOF        ;TURN OFF CLOCK
        XRA         A
        STA         GFLG
        POP         D
        POP         H
        POP         B
        POP         PSW
        RET
        ;
        ;
        ;
        ;
        ;
IRSRV:
        ;
        ;INTERRUPT SERVICE ROUTINE
        ;
        PUSH        PSW
        PUSH        B
        PUSH        H
        PUSH        D
        CALL        RTICK       ;SERVICE INTERRUPT
        LDA         INTFLG
        IF ( .A, IS, NZERO)         ;THEN INTERRUPT INTERRUPT
          NOP
        ELSE
          CMA
          STA       INTFLG      ;SET FLAG
        ENDIF
        ;
        ;
        LHLD        ELPTM
        INX         H
        SHLD        ELPTM
        LDED        SFREQ
        CALL        TDHE
        IF (PSW, IS, ZERO)       ;THEN 1 SECON
          LXI       H, 0
          SHLD      ELPTM
          LHLD      SECONDS
          INX       H
          SHLD      SECONDS
          XRA       A
```

```
        CMA
        STA             SECFLG
        LXI             D, 60
        CALL            TDHE
        IF (PSW, IS, ZERO)      ;THEN 1 MINUTE
            LXI         H, 0
            SHLD        SECONDS
            LHLD        ELPMIN
            INX H
        SHLD            ELPMIN
   ;IF COLLECTING BASELINE: THEN STOP AT 5 MINUTES
            LDA BSEFLG
            IF ( .A, IS, NZERO)
                LXI     D, 5
                LHLD    ELPMIN
                CALL    TDHE
                IF (PSW, IS, ZERO)
                        JMP         CLKSTP
                ENDIF
            ELSE
    ;
    ;
    ;WHILE CALIBRATING: IF REACH 10 MINUTES BEFORE 500 DELTAS,
    ;THEN AUTOMATICALLY STOP
                LXI     D, 10
                LHLD    ELPMIN
                CALL    TDHE
                IF (PSW, IS, ZERO)      ;HAVE REACHED 10 MINUTES
                    JMP     CLKSTP
            ENDIF
            ENDIF
    ;
        ENDIF
    ENDIF
    ;
    ;
    ;
    ;
    ;ALWAYS COLLECT RC INTO RCBUFF,
    ;               AB INTO ABBUFF,
    ;
    ;NOTE: ALWAYS USES DATA IN SPBUFF FOR DETECTION
    ;
        LHLD            RCAD        ;RIB CAGE VALUE
        MOV             A, H
        CMA
        ANI             0FH
        MOV             H, A
        MOV             A, L
        CMA
        MOV             L, A
        SHLD            DATA
        SHLD            RCV
```

```
;
        LHLD            RIPT
        LXI             D, RCBUFF
        CALL            PRNG
;
;

        LHLD            ABAD        ;ABDOMEN VALUE
        MOV             A, H
        CMA
        ANI             0FH
        MOV             H, A
        MOV             A, L
        CMA
        MOV             L, A
        SHLD            DATA
        SHLD            ABV
;
        LHLD            RIPT
        LXI  ·          D, ABBUFF
        CALL            PRNG
;
;SUM INTO SPBUFF--- TO PICK OFF BREATHS
        LHLD            RCV
        LDED            ABV
        DAD             D
        SHLD            DATA
;
        LHLD            RIPT
        LXI             D, SPBUFF
        CALL            PRNG
;
;
;
;
;
;
;INCREMENT RIPT FOR NEXT TIME
;
        LHLD            TIME
        INX             H
        SHLD            TIME
        SHLD            DATA
        LHLD            RIPT
        LXI             D, TMBUFF
        CALL            PRNG
;
        LHLD            RIPT
        SHLD            ODRIPT
;
        LXI             B, RSIZE
        LHLD            RIPT
        CALL            PTRUPD
        SHLD            RIPT
```

21

```
;
  LDA                WRAPF
  IF ( .A, IS, ZERO)      ;SEE IF RIPT RESET TO 0
    LXI              D, 0
    LHLD             RIPT
    CALL             TDHE
    IF (PSW, IS, ZERO)
      MVI A, OFFH
      STA WRAPF
    ENDIF
  ENDIF
;
  LDA                BSEFLG
  IF ( .A, IS, ZERO)
  LDA                ISOFLG
  IF ( .A, IS, NZERO)      ;THEN NO PEAK DETECTION
    JMP              EXIT
  ENDIF
  ENDIF
;
;
  LDA                SLOPEF
  IF ( .A, IS, ZERO)  ;THEN INIT POINTS NOT COLLECTED YET
    CALL             GETP2   ;RETURNS C IF < 5 PTS IN BUFFER
                             ;THIS JUST MAKES SURE EXTRA
                             ;POINTS IN BUFFER
    JC               EXIT    ;NOT ENOUGH POINTS IN BUFFER
;
;NOTE: GETP2 INITIALLIZES ROPT TO LINE UP WITH DERIV STUFF
;SKIP 1ST 3 POINTS IN BUFFER

;   LXI H,6          ;OFFSET POINTING TO POINT 4
;   SHLD             ROPT

;
;
    MVI              A, OFFH
    STA              SLOPEF
  ENDIF
;
;
;
;
  CALL               DERIV5
;
;
;*********************************
;
;MUST ADD THE CODE TO DO PEAK DETECTION
;HERE TOO !!
;
  LDA                FRSTMX
  IF ( .A, IS, ZERO)      ;HAVE NOT FOUND 1ST MAX POINT
;
```

```
        ;
        ;
        ;
            LHLD        ROPT
            CALL        FIND
            IF (PSW, IS, NCARRY)     ;NOT A MAX
                JMP     OUTER
            ENDIF
        ;HAVE 1ST MAX
            LDA         FRSTMX
            CMA
            STA         FRSTMX
            SHLD        SPTEMP      ;TEMP CRITICAL POINT
            SDED        RCTEMP
            SBCD        ABTEMP
            LDA         PTF
            CMA
            STA         PTF       ;NOW LOOK FOR MIN
            JMP         OUTER  .
        ENDIF
        ;
        ;
            LHLD        ROPT
            CALL        FIND
        IF (PSW, IS, CARRY)
            SHLD        SPVALUE
            SDED        RCVALUE
            SBCD        ABVALUE
            LDA         PTF
            IF ( .A, IS, ZERO)       ;THIS IS A MAX POINT
                LHLD    RCVALUE
                SHLD    RCMXVAL
                LHLD    ABVALUE
                SHLD    ABMXVAL
        ;
                CALL    DLTSTORE
        ;ONLY STORE THE MCA VARIABLES AT A MAX (ONLY WANT FOR INSP)
                CALL    MCASTORE
        ;
        ;
        ;IF DLTIPTR = 1000, THEN EXIT
                LHLD    DLTIPTR
                LXI     D, 1000
                CALL    TDHE
                IF (PSW, IS, ZERO)
                    MVI     A, 0FFH
                    STA     DLTFULL
                    LXI     H, 0
                    SHLD    DLTIPTR
                    JMP     CLKSTP
                ENDIF
            ELSE              ;THIS IS MIN POINT
                LHLD    RCVALUE
                SHLD    RCMNVAL
```

23

```
                LHLD    ABVALUE
                SHLD    ABMNVAL
                LDA     FSTMN
                IF ( .A, IS, NZERO)      ;THIS IS THE 1ST MIN, DO NOT STORE
                    LDA     FSTMN
                    CMA
                    STA     FSTMN
                ELSE    ;STORE THE DELTA
                    CALL    DLTSTORE
        ;
        ;
        ;IF DLTIPTR = 1000, THEN EXIT
                    LHLD    DLTIPTR
                    LXI     D, 1000
                    CALL    TDHE
                    IF (PSW, IS, ZERO)
                        MVI   A, 0FFH
                        STA   DLTFULL
                        LXI   H, 0
                        SHLD DLTIPTR
                        JMP   CLKSTP
                    ENDIF
                ENDIF
        ;
            ENDIF
            LDA             PTF
            CMA
            STA             PTF
        ENDIF
        ;
        ;********************************
        OUTER:
        ;
        ;UPDATE ROPT FOR NEXT TIME
        ;
            LHLD            ROPT
            LXI             B, RSIZE
            CALL            PTRUPD
            SHLD            ROPT

        ;
        EXIT:
        ;LOOK FOR ENTER
        ;
        CALL            CSTS
        IF (PSW, IS, NZERO)
            CALL            CINP
            IF ( .A, EQ, 0DH)       ;IF ENTER
                JMP     CLKSTP
            ENDIF
        ENDIF
        ;
        XRA             A
```

```
        STA             INTFLG
        POP             D
        POP             H
        POP             B
        POP             PSW
        RET
        ;
        ;
        ;
        MCASTORE:
        LHLD            MCAADDR
        LDED            RCINVOL
        MOV             M, E
        INX             H
        MOV             M, D
        INX             H
        LDED            ABINVOL
        MOV             M, E
        INX             H
        MOV             M, D
        INX             H
        LDED            SMVOL
        MOV             M, E
        INX             H
        MOV             M, D
        INX             H
        SHLD            MCAADDR
        RET
        ;
        ;
        ;
        ;
        DLTSTORE:
                LHLD    RCMXVAL
                LDED    RCMNVAL
                ORA     A
                DSBC    D
        ;HL = INSP VOLUME;RC
                SHLD    RCVALUE     ;DELTA RC
                LHLD    DLTIPTR     ;OFFSET TO STORE
                LXI     D, RCDATA
                DAD     D
                LDED    RCVALUE
                MOV     M, E
                INX     H
                MOV     M, D
        ;
                LHLD    ABMXVAL
                LDED    ABMNVAL
                ORA     A
                DSBC    D
        ;HL = INSP VOLUME;AB
                SHLD    ABVALUE     ;DELTA AB
                LHLD    DLTIPTR     ;OFFSET TO STORE
                LXI     D, ABDATA
```

```
                  DAD       D
                  LDED      ABVALUE
                  MOV       M, E
                  INX       H
                  MOV       M, D
                  LHLD      DLTIPTR
                  INX       H
                  INX       H
                  SHLD      DLTIPTR
                  LHLD      MAXCNT
                  INX       H
                  SHLD      MAXCNT
                  XRA       A
                  CMA
                  STA       BFLG
         ;STORE THE TIMES
             LHLD            TMPTR
             LXI             D, DLTEND
             CALL            TDHE
         IF (PSW, IS, ZERO)         ;NO ROOM
             RET
             ENDIF
         ;
             LHLD            TMPTR
             LDED            CPTIME        ;TIME OF THE CP
             MOV             M, E
             INX             H
             MOV             M, D
             INX             H
             LDED            PKVALUE       ;TIME OF THE PEAK FLOW
             MOV             M, E
             INX             H
             MOV             M, D
             INX             H
             SHLD            TMPTR         ;FOR NEXT TIME
                             RET
         ;
         ;
         PRNG:
         ;
         ;HL HOLDS THE RING INPUT PTR
         ;DE HOLDS ADDR OF RING
         ;
             DAD       D
             LDED      DATA
             MOV       M, E
             INX       H
             MOV       M, D
             RET
         ;
         ISOMESS:          .ASCIS 'Isovolume'
         ;
         BSEMESS:          .ASCIS 'Baseline'
```

26

```
;
CLBMESS:          .ASCIS 'Calibrating'
BLANKS:           .ASCIS      '    '
;
RES:      .ASCIS '
AT əl:əl:əl   Cal factors reset to 1 and 1
'
RESPT: .WORD    HOUR, MIN, SEC
;
          .EXTERN  QDCOM
          .LOC     QDCOM
;
MAXB: .BLKB    1
RCCU: .BLKB    2
ABCU: .BLKB    2
SPCU: .BLKB    2
RCV:  .BLKB    2
ABV:  .BLKB    2
MEAN: .BLKB    4
FCNT: .BLKB    4
;
FRSTMX:           .BLKB  1
RCMXVAL:          .BLKB  2
RCMNVAL:          .BLKB  2
ABMXVAL:          .BLKB  2
ABMNVAL:          .BLKB  2
FSTMN:   .BLKB  1
FLSHFLG:          .BLKB  1
ISOFLG:           .BLKB  1
XCNT:   .BLKB   4
QDCEXTRA:         .BLKB  9      ;EXTRA LOCS
          .RELOC
          .END     QDC
;
;
;
;
```

EP 0 256 115 B1

FILE: QDCAL.ASM

```
    .IDENT     QDCAL
    .INSERT    FPMAC.SRC
    .INSERT    SP80
    FINIT
    .INSERT    VTCCOM
    .INSERT    CALCOM
    .INSERT    OLQCOM
    .INSERT    CKCOM
    .INSERT    VARCOM.CCC
    .INSERT    SYCOM
    .INSERT    EFFCOM
    .INSERT    SIOCOM
    .INSERT    QD1.ASM
    .INSERT    PKCOM
;
    .EXTERN    CMPSTATS
;
    .ENTRY     QDCAL
    .EXTERN    TDHE, ROUND
    .EXTERN    SAVECL, ANSOUT, LPON, LPOFF
    .ENTRY     V2        ;SO CAN LOOK AT MAP!
    .EXTERN    PRTIME
    .EXTERN    WTSAVECL
;
    .EXTERN    TVON, TVOFF
;
;
;
QDCAL:
    LDA        BSEFLG
    IF ( .A, IS, NZERO)        ;BASELINE
        JMP    BCOMP
    ENDIF

;ENTERED HERE, THEN IN CALIBRATE MODE (NOT LONG TERM)
;
    CALL       CMPSTATS
    RC                    ;CARRY SET IF BAD CAL
                          ;BECAUSE NOT ENOUGH DATA COLLECTED
;
;
;ABCAL = RCSTD / ABSTD
    FLDB       RCSTD
    FLDA       ABSTD
    FDIV
    FSTA       ABCAL
;RCCAL = 1
    FFOA       1
    FSTA       RCCAL
;
;
;MUST STORE THE GAINS, BECAUSE RESCALE ASSUMES SO !!
    LDA        WTFLG
```

28

```
        IF ( .A, IS ZERO)        ;WANT TO PRINT ERROR CODE IF BAD
           CALL    SAVECL
        ELSE
           CALL    WTSAVECL
        ENDIF
        IF (PSW, IS, CARRY)        ;THEN BAD CALS
           MVI     A, 0    ;NO GOOD CALIBRATION (WHEN BEGAN THIS RUN, RESET
           STA     CALTYPE ;CALS TO 1 AND 1
           MVI     A, 0
           STA     LASTCAL ;NO GOOD CAL
           STA     GOODBASE        ;NO GOOD BASELINE NOW
        ;PRINT OUT WHAT THE BAD CALS WERE AND THE STDS
           CALL    BDCALS
           STC             ;BAD CALS
           RET
        ENDIF
        ;
        ;
        ;
        ;NOW MUST USE THE MEAN VT MEASURED TO SCALE THE CAL FACTORS SO
        ; MEAN VT = VTMX/10
           CALL    RESCALE
        ;
        LDA     WTFLG
        IF ( .A, IS, ZERO)
           MVI     A, 4
           STA     OPCODE
           CALL    ANSOUT          ;DISPLAY THE CALS ON THE MONITOR
        ENDIF
        ;
        LDA     WTFLG
        IF ( .A, IS, ZERO)        ;WANT TO PRINT ERROR CODE IF BAD
           CALL    SAVECL
        ELSE
           CALL    WTSAVECL
        ENDIF
        IF (PSW, IS, NCARRY)        ;THEN GOOD CALS
           CALL    LPON
           CALL    PRTIME
           FPRN    V1, V1PT
           CALL    LPOFF
           CALL    CMPMCA          ;COMPUTE MCA/VT
           CALL    LPON
           FPRN    V2, V2PT
           CALL    PRTHYP
           CALL    LPOFF
           MVI     A, 1        ;QDC QUAL
           STA     CALTYPE
           MVI     A, 0FFH
           STA     LASTCAL
           ORA     A       ;GOOD CALS
        ELSE
           MVI     A, 0        ;NO GOOD CALIBRATION (WHEN BEGAN THIS RUN, RESET
           STA     CALTYPE         ;CALS TO 1 AND 1
```

```
        MVI     A, 0
        STA     LASTCAL     ;NO GOOD CAL
        STA     GOODBASE        ;NO GOOD BASELINE NOW
;PRINT OUT WHAT THE BAD CALS WERE AND THE STDS
;
        CALL    BDCALS
        FFOA    1
        FSTA    RCCAL
        FSTA    ABCAL
        CALL    CMPMCA      ;COMPUTE MCA/VT
        CALL    LPON
        FPRN    V2, V2PT
        CALL    PRTHYP
        LXI     H, STARS
        CALL    TXTYP
        CALL    LPOFF
;
        STC             ;BAD CALS
        ENDIF
        RET
;
;
;
;
BDCALS:
;
;
        CALL    LPON
        LXI     H, STARS
        CALL    TXTYP
        CALL    PRTIME
        FPRN    BADC, BADPT
        CALL    LPOFF
        RET
;
;
;
PRTHYP:
        FPRN        HYP, HYPPT
        LDA         QUALFLG
        IF ( .A, IS, NZERO)      ;QUAL
          LXI H, RHYP

        ELSE            ;QUANT
          LXI   H, AHYP
        ENDIF
        CALL        TXTYP
        RET
;

BCOMP:
;THIS COMPUTE MEAN VT AND IF ISOV WAS QUALITATIVE, CALTYPE = 3,
;THEN IT SCALES IT TO VTMAX/10
;
```

```
        CALL        CMPSTATS
        IF (PSW, IS, ZERO)        ;NOT ENOUGH DATA
            XRA        A
            STA        GOODBASE        ;NOT A GOOD BASELINE
            STC
            RET
        ENDIF
    ;
        CALL        RESCALE        ;THIS WILL CHANGE VALUE OF RCCAL AND ABCAL
    ;
        LDA        CALTYPE
        IF ( .A, EQ, 3)    ;ISOV QUAL
    ;
    ;SAVE CALS
    ;NOW MUST USE THE MEAN VT MEASURED TO SCALE THE CAL FACTORS SO
    ;MEAN VT = VTMX/10
            CALL        WTSAVECL
    ;
    ;
        IF (PSW, IS, NCARRY)        ;THEN GOOD CALS
            MVI A, 5        ;ISO-QUAL WITH BASELINE
            STA CALTYPE
            MVI A, OFFH
            STA LASTCAL ;GOOD CAL
            CALL        LPON
            CALL        PRTIME
            FPRN        BS1, BS1PT
            CALL        LPOFF
            CALL        CMPMCA    ;COMPUTE MCA/VT
            CALL        LPON
            FPRN        V2, V2PT
        CALL    PRTHYP
            CALL        LPOFF
            MVI A, 0FFH
            STA GOODBASE        ;GOOD BASELINE
            ORA A        ;GOOD CALS
        ELSE
            FFOA        1
            FSTA        RCCAL
            FSTA        ABCAL
            CALL        CMPMCA    ;COMPUTE MCA/VT
            CALL        LPON
            LXI H, STARS
            CALL        TXTYP
            CALL        PRTIME
            FPRN        BSBAD, BSBPT
            FPRN        V2, V2PT
        CALL    PRTHYP
            LXI H, STARS
            CALL        TXTYP
            CALL        LPOFF
            MVI A, 0
            STA GOODBASE        ;BAD BASELINE
```

31

```
        STC             ;BAD CALS
    ENDIF
 ELSE     CALREADY DID QUANT ISOV
 IF ( .A, EQ, 5)
    MVI A, 6        ;ISO QUANT WITH BASELINE
    STA   CALTYPE
        CALL        LPON
        CALL        PRTIME
        FPRN        BS3, BS3PT
        CALL        LPOFF
;CALS DO NOT CHANGE
        FFOA        1
        FSTA        RCCAL
        FSTA        ABCAL
        CALL        CMPMCA  ;COMPUTE MCA/VT
        CALL        LPON
        FPRN        V2, V2PT
    CALL   PRTHYP
        CALL        LPOFF
    MVI A, 0FFH
    STA GOODBASE        ;GOOD BASELINE
    ORA A           ;GOOD CALS
 ELSE
 IF ( .A, EQ, 7)    ;TYPED IN CALS, QUANT, JUST GETTING BASELINE STUFF
    LDA   QUALFLG
    IF ( .A, IS, ZERO)
        CALL        LPON
        CALL        PRTIME
        FPRN        BS7, BS7PT
        CALL        LPOFF
;CALS DO NOT CHANGE
        FFOA        1
        FSTA        RCCAL
        FSTA        ABCAL
        CALL        CMPMCA  ;COMPUTE MCA/VT
        CALL        LPON
        FPRN        V2, V2PT
    CALL   PRTHYP
        CALL        LPOFF
    MVI A, OFFH
    STA GOODBASE        ;GOOD BASELINE
    ORA A       ;GOOD CALS
    ELSE            ;TYPED IN CALS, QUAL SO WILL CHANGE CALS
;**
;
;SAVE CALS
;NOW MUST USE THE MEAN VT MEASURED TO SCALE THE CAL FACTORS SO
;MEAN VT = VTMX/10
    CALL WTSAVECL
;
;
    IF (PSW, IS, NCARRY)         ;THEN GOOD CALS
        CALL            LPON
```

```
        CALL        PRTIME
        FPRN        V7, V7PT
        CALL        LPOFF
        CALL        CMPMCA   ;COMPUTE MCA/VT
        CALL        LPON
        FPRN        V2, V2PT
      CALL  PRTHYP
        CALL        LPOFF
        MVI A, 0FFH
        STA GOODBASE         ;GOOD BASELINE
        ORA A       ;GOOD CALS
      ELSE
        FFOA        1
        FSTA        RCCAL
        FSTA        ABCAL
        CALL        CMPMCA   ;COMPUTE MCA/VT
        CALL        LPON
        CALL        PRTIME
        FPRN        BSBAD, BSBPT
        FPRN        V2, V2PT
      CALL  PRTHYP
        CALL        LPOFF
        MVI A, 0
        STA GOODBASE         ;BAD BASELINE
        STC         ;BAD CALS
      ENDIF
   ;**
      ENDIF
    ENDIF
    ENDIF

    ENDIF
    RET
;
;
;
    CMPMCA:

    ;
    ;
    ;COMPUTE MEAN MCA/VT
    ;MUST REMEMBER THAT ALL RC VALUES MUST BE MULT BY RCCAL
    ;AND ALL AB VALUES MUST BE MULT BY ABCAL
    ;
    ;NOTE: WILL NOT BE ABLE TO COMPUTE MCA/VT THE CLEAN WAY OF USING
    ;        THEN SUM OF THE SUM'S DERIV BECAUSE THE THE CAL FACTOR PROBLEM
    ;        SO WILL HAVE TO DIVIDE BY RC DELTA * RCCAL + AB DELTA * ABCAL
    LXI             H, XVALUES
    SHLD            ADDR1
    LDA             DLTFULL
    IF ( .A, IS, NZERO)       ;FULL
      LHLD          DLTIPTR
      SHLD          OFFSET
```

```
        ELSE
         LXI          H, 0
          SHLD        OFFSET
        ENDIF

        FFOA         0
        FSTA         VTSUM
        FSTA         MCASUM
        FSTA         XCNT
LOOP
        LHLD         ADDRI
        LDED         MCAADDR
        CALL         TDHE
        IF (PSW, IS, ZERO)
        RET
        ENDIF
;
;GET MCA VALUE
        LHLD         ADDP1
        MOV      .   E, M
        INX          H
        MOV          D, M
        SDED         RCMCA
        INX          H
        MOV          E, M
        INX          H
        MOV          D, M
        SDED         ABMCA
        INX          H
        MOV          E, M
        INX          H
        MOV          D, M
        SDED         SMMCA
        INX          H
        SHLD         ADDR1
;
        FILA         RCMCA
        FLOT
        FLDB         RCCAL
        FMUL
        FPSH
        FILA         ABMCA
        FLOT
        FLDB         ABCAL
        FMUL
        FATB
        FPOP
        FADD
        FATB
        FFOA         15      ;BECAUSE OF 9 PT DERIV 60
                            ;/4 BECAUSE OF DIVISION WHEN COLLECTED
        FDIV
        FLDB         CCS
```

```
        FMUL
        FSTA                MCAVALUE
   ;
   ;GET VT VALUE
   LHLD                OFFSET
   LXI                 D, RCDATA
   DAD                 D
   MOV                 E, M
   INX                 H
   MOV                 D, M
   SDED                FRC
   ;
   LHLD                OFFSET
   LXI                 D, ABDATA
   DAD                 D
   MOV                 E, M
   INX                 H
   MOV                 D, M
   SDED                FAB
   ;
   FILA                FRC
   FLOT
   FLDB                RCCAL
   FMUL
   FPSH
   FILA                FAB
   FLOT
   FLDB                ABCAL
   FMUL
   FATB
   FPOP
   FADD
   FLDB                CCS
   FMUL
   FSTA                VTVALUE
   ;
   ;
   ;
   MCASUM = SUM OF MCAVALUE/VTVALUE
   ;
   FLDB                MCAVALUE
   FLDA                VTVALUE
   FDIV
   ;IF < 1, THEN MAKE = 1
   FPSH
   FATB
   FFOA                1
   FSUB
   FTST                AREG, LT
   IF (PSW, IS, ZERO)
      FPOP
      FFOA             1
   ELSE
      FPOP
```

35

```
        ENDIF
    ;
        FLDB                MCASUM
        FADD
        FSTA                MCASUM
    ;
        FLDB                VTVALUE
        FLDA                VTSUM
        FADD
        FSTA                VTSUM

        FLDB                XCNT
        FFOA                1
        FADD
        FSTA                XCNT
    ;
        LHLD                OFFSET
        INX                 H
        INX                 H
        SHLD        .       OFFSET
        LDED                DLTIPTR
        CALL                TDHE
        IF (PSW, IS, ZERO)
            RET
        ENDIF
    ;
    ;NOW OFFSET POINTS TO EXP DELTA — SKIP IT !!
    ;
        LHLD                OFFSET
        INX                 H
        INX                 H
        SHLD                OFFSET
        LDED                DLTIPTR
        CALL                TDHE
        IF (PSW, IS, ZERO)
            RET
        ENDIF
    ENDLOOP
    ;
    ;
        FLDB                VTSUM
        FLDA                XCNT
        FDIV
        FSTA                VTVALUE         ;MEAN VT
    ;
        FLDB                MCASUM
        FLDA                XCNT
        FDIV
        FSTA                MCAVT           ;MEAN MCA/VT
    ;
    ;
    ;MEAN MCA/VT = MEAN MCA / MEAN VT
    ;
```

```
;    FLDB              MCAVALUE
;    FLDA              VTVALUE
;    FDIV
;;IF   1, THEN SET = 1
;    FPSH
;    FATB
;    FFOA              1
;    FSUB
;    FTST              AREG, LT
;    IF (PSW, IS, ZERO)
;        FPOP
;        FFOA          1
;    ELSE
;        FPOP
;    ENDIF
;    FSTA              MCAVT
;
;
;
;COMPUTE THE MEAN VALUE OF % TIME TO PEAK INSP FLOW
;AND MEAN VALUE OF % TIME TO PEAK EXP FLOW
     LHLD              TMPTR
     LXI               D, DLTIMES
     CALL              TDHE
     IF (PSW, IS, ZERO)        ;NONE
         LXI           H, 0
         SHLD          PCNUMBERS
         RET
     ENDIF
;
     FFOA              0
     FSTA              PKISUM
     FSTA              PKESUM
     FSTA              TISUM
     LXI               H, 0
     SHLD              PCNUMBERS
     LXI               H, DLTIMES
     SHLD              ADDR1
     LOOP
;MAKE SURE THAT ADDR1 + 10 < TMPTR
     LHLD              ADDR1
     LXI               D, 10
     DAD               D
     LDED              TMPTR
     ORA               A
     DSBC              D
     IF (PSW, IS, NCARRY)          ;THEN ALL DATA NOT THERE
         RET
     ENDIF
;
;ASSUME THAT ADDR1 PTS TO MIN1
     LHLD              ADDR1
     MOV               E, M
```

```
        INX             H
        MOV             D, M
        SDED            TMIN1
    ;GET PKE, MAX, PKI, MIN2 VALUES
        INX             H
        MOV             E, M
        INX             H
        MOV             D, M
        SDED            PKE         ;FOR LAST BREATH
        INX             H
        MOV             E, M
        INX             H
        MOV             D, M
        SDED            TMAX
        INX             H
        MOV             E, M
        INX             H
        MOV             D, M
        SDED            PKI
        INX             H
        SHLD            ADDR1       ;SO NEXT TIME ADDR1 PTS TO MIN1 OF NEXT
        MOV             E, M
        INX             H
        MOV             D, M
        SDED            TMIN2
        INX             H
        MOV             E, M
        INX             H
        MOV             D, M
        SDED            PKE         ;FOR THIS BREATH
    ;% T TO PEAK INSP FLOW = (PKI-TMIN1) / (TMAX-TMIN1) + 100%
        LHLD            TMAX
        LDED            TMIN1
        ORA             A
        DSBC            D
        SHLD            TTEMP
        LHLD            PKI
        LDED            TMIN1
        ORA             A
        DSBC            D
        SHLD            TT2EMP
        FILA            TT2EMP
        FLOT
        FATB
        FILA            TTEMP
        FLOT
        FDIV
        FATB
        FFOA            100
        FMUL
    ;% T TO PEAK INSP FLOW
        FLDB            PKISUM
        FADD
```

```
        FSTA              PKISUM
     ;% T TO PEAK EXP FLOW: = (PKE-TMAX) / (TMIN2-TMAX) + 100%
        LHLD              PKE
        LDED              TMAX
        ORA               A
        DSBC              D
        SHLD              TTEMP
        LHLD              TMIN2
        LDED              TMAX
        OPA               A
        DSBC              D
        SHLD              TT2EMP
        FILA              TTEMP
        FLOT
        FATB
        FILA              TT2EMP
        FLOT
        FDIV
        FATB
        FFOA·             100
        FMUL
     ;% T TO PEAK EXP FLOW
        FLDB              PKESUM
        FADD
        FSTA              PKESUM
     ;
     ;TI = TMAX — TMIN1 / SFREQ
        LHLD              TMAX
        LDED              TMIN1
        ORA               A
        DSBC              D
        SHLD              TTEMP
        FILA              TTEMP
        FLOT
        FATB
        FILA              SFREQ
        FLOT
        FDIV
     ;AREG = TI'
        FLDB              TISUM
        FADD
        FSTA              TISUM

     ;
        LHLD              PCNUMBERS
        INX               H
        SHLD              PCNUMBERS

        ENDLOOP
     ;
        FLDB              PKISUM
        FILA              PCNUMBERS
        FLOT
```

39

```
        FDIV
        FSTA            PKIAVG
    ;
        FLDB            PKESUM
        FILA            PCNUMBERS
        FLOT
        FDIV
        FSTA            PKEAVG
    ;
        FLDB            TISUM
        FILA            PCNUMBERS
        FLOT
        FDIV
        FSTA            TICONTROL
    ;
    ;VTTICONTROL = ONEHUND / TI
        FFOP
        FATB            100      ;%

        FLDA    .       TICONTROL
        FDIV
        FSTA            VTTICONTROL


    ;
    ;NOTE: THIS COMPUTATION MUST BE DONE AFTER MCA COMPUTATION
    ;       BECAUSE HYPOPV AND MCAVALUE ARE AT THE SAME ADDR
    ;       TO CONSERV RAM !!!!
    ;
    ;WILL WANT TO PRINT OUT VALUE OF HYPOPNEA (VTLIMIT) = ML
        LDA             QUALFLG
        IF ( .A, IS, ZERO)      ;QUANT SO IN ML
            FLDA        VTLIMIT
            FSTA        HYPOPV        ;HYPOP IN ML
        ELSE                    ;QUAL SO PRINT IN TERMS OF %
            FLDB        VTMX
            FFDA        10
            FDIV
    ;AREG = CONTROL VALUE IN ML
            FLDB        VTLIMIT       ;HYPOP VOLUME IN ML
            FDIV
            FATB
            FFOA        100
            FMUL
            FSTA        HYPOPV        ;HYPOP IN % OF CONTROL
        ENDIF
    ;
    ;
        RET
    ;



    ;
    STARS: .ASCIS '
    ***********************************
```

```
'
;
;
;
BSBAD: .ASCIS'
**BAD ISOVOLUME SCALING CALIBRATION:
    TRYED TO SCALE TO ∂D3 ML

    RETRY WITH Y-SCALING AT LOWER VALUE '
;
BSBPT: .WORD   ONEHUND
;
;
;
;
BADC: .ASCIS'
*** BAD QUALITATIVE CALIBRATION: ***
    RC STD    = ∂D3
    AB STD    = ∂D3
    # DELTAS = ∂D1
    MEAN RC  = ∂D3
    MEAN AB  = ∂D3
'
;
;
BADPT:

.WORD       RCSTD, ABSTD, XCNT, FRC, FAB
;
;
;
;
;
;
;
BS1:       .ASCIS'
BASELINE:
QUALITATIVE ISOVOLUME CALIBRATION:
    From ∂D1 deltas:
          RC CAL = ∂D3
          AB CAL = ∂D3
    Breathing Freq = ∂D3 Bths/min
'
;
;
BS3:       .ASCIS'
BASELINE WITH QUANTITATIVE ISOVOLUME CALIBRATION:
    Breathing Freq = ∂D3 Bths/min
'
;
BS3PT:
  .WORD   FREQCONTRL
;
BS7:       .ASCIS'
BASELINE WITH GAINS TYPED IN QUANTITATIVELY
    Breathing Freq = ∂D3 Bths/min
```

```
;
;
BS7PT:
  .WORD  FREQCONTRL
;
;
;
V1:  .ASCIS'
QUALITATIVE CALIBRATION:
  From ∂D1 deltas:
          RC CAL = ∂D3
          AB CAL = ∂D3
  Breathing Freq = ∂D3 Bths/min
,
;
;
;
V7:  .ASCIS'
BASELINE WITH GAINS TYPED IN QUALITATIVELY:
  From ∂D1 deltas:
          RC CAL = ∂D3
          AB CAL = ∂D3
  Breathing Freq = ∂D3 Bths/min
,
;
;
;
V2:  .ASCIS'
  From ∂D1 breaths:
  Mean MCA/VT = ∂D3
  Mean %T PK INSP FL = ∂D3 %
  Mean %T PK EF FL = ∂D3 %
  Mean TI = ∂D3 sec'
;

HYP:  .ASCIS'
  Hypopnea volume = ∂D3'
AHYP:  .ASCIS 'ml
,

RHYP:  .ASCIS '% of baseline
,
HYPPT:  .WORD  HYPOPV
;
;
;
;
BS1F
V7PT:
V1PT:  .WORD  XCNT, RCCAL, ABCAL
       .WORD  FREQCONTRL
;
V2PT:  .WORD  XCNT
       .WORD  MCAVT
```

```
        .WORD   PKIAVG, PKEAVG
        .WORD   TICONTROL

;
;
;
;
RESCALE:
    FLDB        VTMX
    FFOA        10
    FDIV
    FSTA        ONEHUND   ;THIS THE THE VOLUME SCALING TO
;COMPUTE THE FREQ SO CAN COMPUTE THE VMIN FOR CONTROL
    FILA        SECONDS
    FLOT
    FATB
    FFOA        60
    FDIV
    FATB
    FILA .      ELPMIN
    FLOT
    FADD        ;AREQ = # MINUTES FOR COLLECTION
    FPSH
;
    LHLD        MAXCNT   ;# OF DELTAS
    SRAR        H
    RARR        L        ;MAXCNT/2 == # BREATHS
    SHLD        ITEMP
    FILA        ITEMP
    FLOT
    FATB        ;# BREATHS
    FPOP        ;# MINUTES
    FDIV
    FSTA        FREQCONTRL
;
    FLDB        ONEHUND   ;100 % VOLUME
    FLDA        FREQCONTRL      ;FREQ
    FMUL
    FSTA        VMINCONTRL      ;VMIN OF CONTROL
;
;
;
;
;SMMEAN = MEAN VT IN CUS
    FLDB        SMMEAN
    FLDA        CCS
    FMUL
    FSTA        MEAN     ;MEAN VT IN ML
;
;
    LDA         QUALFLG
    IF ( .A, IS, NZERO)      ;QUALITATIVE
;
```

```
;SO INIT VTLIMIT TO ONEH ND + VTPCL%
        FLDB        VTPCL
        FFOA        100
        FDIV
        FLDB        ONEHUND
        FMUL
        FSTA        VTLIMIT        ;FOR HYPOPNEA DETECTION
    ;
        ELSE                        ;QUANTITATIVE, USE MEAN VT INSTEAD OF ONEHUND
        FLDB        VTPCL
        FFOA        100
        FDIV
        FLDB        MEAN        ;MEAN VT IN ML
        FMUL
        FSTA        VTLIMIT        ;FOR HYPOPNEA DETECTION
        ENDIF
    ;
        FLDB        VTMX
        FFOA        10
        FDIV
        FLDB        MEAN
        FSWP
        FDIV            ;RATIO OF (VTMX/10) / MEAN VT
        FSTA        MEAN
;RCCAL = CURRENT RC GAIN (RCGNF) + MEAN
;ABCAL = CURRENT AB GAIN (HBGNF) + MEAN
        FLDA        MEAN
        FLDB        RCGNF        ;CAL FROM INITIAL CALCS
        FMUL
        FSTA        RCCAL
    ;
        FLDA        MEAN
        FLDB        ABGNF        ;CAL FROM INITIAL CALCS
        FMUL
        FSTA            ABCAL
    ;
;NOW THE CAL FACTORS ARE SCALED SO VTMX/10 WILL APPEAR AB 100% DURING
;LONG TERM
        RET

        .EXTERN QD2
        .LOC        QD2
RCMCA: .BLKB 2
ABMCA: .BLKB 2
SMMCA: .BLKB 2
ADDR1: .BLKB  2
    ;
MCAVALUE:
HYPOPV:
        .BLKB    4
    ;
    ;
    ;
VTVALUE:        .BLKB 4
MCAVT: .BLKB  4
VTSUM: .BLKB  4
MCASUM:        .BLKB 4
PKIAVG:        .BLKB 4
PKEAVG:        .BLKB 4
TISUM: .BLKB  4
        .RELOC
    ;
        .END
```

```
FILE:   USETUP.ASM
        .IDENT  VSETUP
        .INSERT SP80.ASM
        .INSERT FPMAC.SRC
        .INSERT CALCOM.ASM
        .INSERT CKCOM
        .INSERT SELCOM
        .INSERT BAGCOM
        .INSERT VARCOM.CCC
        .INSERT SYCOM
        .INSERT HRDCOM
;
        .EXTERN APBP, ZALL, AUDEN
;
        .EXTERN RTON, RTOF, RTICK
        .EXTERN TDHE
        .EXTERN DERIV5
        .EXTERN PTRUPD, GETP2, FIND, ROUND
;
        .EXTERN SPON, SPOF
;
;
        .ENTRY  SETUP
;
;
CR = 0DH
    FINIT
;
;
SETUP:
;
;SETUP THE CLOCK FOR VALIDATION
;
;SAMPLE AT 20 PTS PER SEC
    PUSH        H
;INIT REAL-TIME BUFFER POINTERS
;MODIFIED 09-24-81
    MVI         A, 3
    STA         WAITFLG
;
    XRA         A
    STA         WRAPF
    STA         SECFLG
    CMA
    STA         BFLG
    XRA         A
    STA         VFRSTMX
    LXI         H, 0
    SHLD        ELPTM
    CHLD        ELPMIN
    SHLD        SECONDS
    SHLD        CPPTR
;
```

```
        LXI       H, 0
        SHLD      RIPT
        SHLD      ODRIPT
        SHLD      SPTEMP
        SHLD      PNSUM
    ;
    ;
    ;
        LXI       H, 8      ;SKIP THE 1ST 4 DATA POINTS
        SHLD      ROPT
    ;
        LXI       H, TMBUFF
        MVI       M, 0
        INX       H
        MVI       M, 0
        LXI       H, 0
        SHLD      TIME
    ;INIT VARIABLES FOR PICKING MAX AND MINS
        XRA       A
        STA       PTF       ;START BY LOOKING FOR MAX PT
        STA       SLOPEF    ;INIT MAX NOT FOUND
    ;
        XRA       A
        STA       INTFLG
        CMA
        STA       GFLG      ;INDICATES CLOCK ON
        CALL      AUDEN
        CALL      APBP
        CALL      ZALL
    ;
    ;
        LXI       H, IRSRV
        MVI       A, 20     ;CLOCK RATE
        CALL      RTON      ;TURN ON CLOCK
    ;
        LDA       BAG
        IF (.A, IS, ZERO)   ;USING SPIROMETER
          CALL    SPON
        ENDIF
    ;
    ;
        POP       H
        RET
    ;
    ;
    ;
CLKSTP:
    ;STOP DATA COLLECTION
    ;TURN CLOCK OFF
    ;
;MODIFIED 09-24-81
    ;
        CALL      RTOF      ;TURN OFF CLOCK
        LDA       BAG
```

46

```
        IF ( .A, IS, ZERO)    ;USING SPIROMETER
           CALL    SPOF
        ENDIF
        XRA     A
        STA     GFLG
        CALL    AUDEN
        CALL    APBP
;********************
        POP     PSW
        POP     H
        POP     D
        POP     B
;
        RET
;
;
;
    IRSRV:
;
;INTERRUPT SERVICE ROUTINE
;
        PUSH    B
        PUSH    D
        PUSH    H
        PUSH    PSW
;
;
        CALL    RTICK        ;SERVICE INTERRUPT
        LDA     INTFLG
        IF (.A, IS, NZERO)        ;THEN INTERRUPT INTERRUPT
           NOP
        ELSE
           CMA
           STA     INTFLG      ;SET FLAG
        ENDIF
;
        CALL    CLKTIK
;
;
;
;
        CALL    SAMPLE
;
;
;
;
        LDA     SLOPEF
        IF ( .A, IS, ZERO)
           CALL    GETP2      ;RETURNS C IF < 5 PTS IN BUFFER
                              ;THIS JUST MAKES SURE EXTRA
                              ;POINTS IN BUFFER
           JC      EXIT     ;NOT ENOUGH POINTS IN BUFFER
```

47

```
        MVI     A, OFFH
        STA     SLOPEF
      ENDIF
  ;
  CALL        DERIV5
  ;
  LDA         VFRSTMX
  IF ( .A, IS, ZERO)      ;HAVE NOT FOUND 1ST MAX
    LHLD      ROPT
    CALL      FIND
    IF (PSW, IS, NCARRY)      ;NOT MAX
              LHLD ROPT
              LXI   B, RSIZE
              CALL  PTRUPD
              SHLD ROPT
              JMP   EXIT
    ENDIF
              ;MAX FOUND
    LDA       VFRSTMX
    CMA
    STA       VFRSTMX
    LDA       PTF
    CMA
    STA       PTF
    SHLD      SPTEMP   ;TEMP CRIT PT
    SDED      RCTEMP   ;CORRESPONDING RC PT
    SBCD      ABTEMP   ;CORRESPONDING AB PT
  ;
    LHLD      ROPT
    LXI       B, RSIZE
    CALL      PTRUPD
    SHLD      ROPT
    JMP       EXIT
  ENDIF
  ;
  ;
              ;INIT MAX FOUND
    LHLD      ROPT
    CALL      FIND
  ;
;UPDATE ROPT FOR NEXT TIME
  ;
    PUSH      PSW
    PUSH      D
    PUSH      B
    PUSH      H
  ;
    LHLD      ROPT
    LXI       B, RSIZE
    CALL      PTRUPD
    SHLD      ROPT
    POP       H
    POP       B
```

```
        POP     D
        POP     PSW
    ;
    ;
    ;FIND RETURNED C SET IF CP FOUND
    ;AND HL HOLDS SP PT VALUE
    ;AND DE HOLDS RC PT VALUE
    ;AND BC HOLDS AB PT VALUE
    ;
    IF (PSW, IS, CARRY)
        SHLD    SPVALUE
        SDED    RCVALUE
        SBCD    ABVALUE
    ;
    ;
        LDA     PTF
        IF ( .A, IS, ZERO)   ;THEN MAX FOUND
                XRA     A
                CMA
                STA     BFLG
                LXI     D, SPMAX
                LHLD    CPPTR
                DAD     D
    ;
                LDA     BAG
                IF ( .A, IS, ZERO)   ;THEN USING SPIROMETER, AND
                                     ;SPVALUE HOLDS SPIROMETER VALUE
                LDED    SPVALUE
                ELSE            ;USING SPIROBAG, SO FIXCU HOLDS VALUE
                LDED    FIXCU
                ENDIF
    ;
                MOV     M, E
                INX     H
                MOV     M, D
    ;
                LHLD    CPPTR
                LXI     D, RCMAX
                DAD     D
                LDED    RCVALUE
                MOV     M, E
                INX     H
                MOV     M, D
    ;
                LHLD    CPPTR
                LXI     D, ABMAX
                DAD     D
    ;
    ;
    ;IF VALIDATING AND IF USING SPIROBAG
    ;THEN SPVALUE HOLDS THE SUM MAX
                LDA     BAG
                IF ( .A, IS, NZERO)      ;THEN USING SPIROBAG
```

```
            LDED    SPVALUE         ;SUM VALUE
            JMP     L1001
            ENDIF
    ;
            LDED    ABVALUE
    L1001:
            MOV     M, E
            INX     H
            MOV     M, D
            LHLD    MAXCNT
            INX     H
            SHLD    MAXCNT
            LHLD    CPPTR
            INX     H
            INX     H
            SHLD    CPPTR
            LHLD    MAXCNT
            LXI     D, BRTHMX
            ORA     A
            DSBC    D
            IF (PSW, IS, ZERO)      ;THEN BUFFER FULL
                JMP     CLKSTP
            ENDIF
        ELSE
            LHLD    CPPTR
            LXI     D, SPMIN
            DAD     D
    ;
            LDA     BAG
            IF ( .A, IS, ZERO)   ;THEN USING SPIROMETER, AND
                                 ;SPVALUE HOLDS SPIROMETER VALUE
            LDED    SPVALUE
            ELSE                 ;USING SPIROBAG, SO MIN = 0
            LXI     D, 0
            ENDIF
    ;
            MOV     M, E
            INX     H

            MOV     M, D
    ;
            LHLD    CPPTR
            LXI     D, RCMIN
            DAD     D
            LDED    RCVALUE
            MOV     M, E
            INX     H

            MOV     M, D
    ;
            LHLD
           ·LXI     D, ABMIN
            DAD     D
    ;
```

```
            LHLD    DATA
            MOV     A, H
            CMA
            ANI     0FH
            MOV     H, A
            MOV     A, L
            CMA
            MOV     L, A
            SHLD    DATA
;
    ENDIF
    RET
;
;
;
;
;
;
;
    PRNG:
;
    ;HL HOLDS THE RING INPUT PTR
    ;DE HOLDS ADDR OF RING
    ;
    DAD     D
    LDED    DATA
    MOV     M, E
    INX     H
    MOV     M, D
    RET
;
    .END
```

```
FILE:  QDCSTATS.ASM
    .IDENT  QDSTATS
    .INSERT      FPMAC.SRC
    .INSERT SP80    FINIT
    .INSERT      VTCCOM
    .INSERT      CALCOM
    .INSERT      OLQCOM
    .INSERT      CKCOM
    .INSERT      VARCOM.CCC
    .INSERT      SYCOM
    .INSERT      QD1.ASM
;
;
;
    EXTERN TDHE, ROUND
;
;
;
    .ENTRY       PRTQDC
    .ENTRY       CMPSTATS
;
PRTQDC:  ·
;  THIS IS THE ENTRY POINT FROM LONG TERM
    CALL       CMPSTATS
    RC
    FLDB       RCSTD
    FLDA       ABSTD
    FDIV
    FSTA       ABCAL
    FPRN       QDCV, QDCPT
    RET
;

QDCV:  .ASCIS'
QDC = ∂D3;      # DELTAS = ∂D1
    MEAN  STD   COV
RC:  ∂D3   ∂D3    ∂D3
AB:  ∂D3   ∂D3    ∂D3´
;
QDCPT: .WORD     ABCAL, XCNT
       .WORD     .FRC, RCSTD, COVRC
       .WORD     FAB, ABSTD, COVAB
;
;
NODELTS:          .ASCIS'
NO QDC DELTAS AVAILABLE
'

;
;
;
;
;
CMPSTATS:
;RCDATA AND ABDATA HOLD THE DELTAS FOR RC AND AB
;
;COMPUTE THE MEAN AND STD OF RC, AB AND RC+AB
;
```

```
        LHLD            DLTIPTR
        SHLD            DLTOFF   ;SAVE IT INCASE IT CHANGES IN THE
                                 ;MIDDLE OF THIS DURING LONG TERM
    ;
    ;
        LDA             DLTFULL
        IF ( .A, IS, ZERO)  ;RING NOT FULL YET
          LXI           H, 0
          SHLD          OFFSET
          LDED          DLTOFF
          CALL          TDHE
          IF (PSW, IS, ZERO)      ;THEN NO DELTAS
                        LXI    H, NODELTS
                        CALL   TXTYP
                        STC
                        RET
          ENDIF
        ELSE                     ;RING IS FULL
          LHLD            DLTOFF       ;POINTS PTO NEXT PLACE TO PUT, SO WILL BE
                                       ;THE OLDEST VALUE
          SHLD  OFFSET
        ENDIF
    ;
    ;
    ;
    ;1ST TIME THRU THE DATA COMPUTE MEAN AND STD OF JUST SUM
    ;
    ;2ND TIME THUR THE DATA ONLY USE DATA WHERE SUM IS WITHIN +-QDCSTD OF MEAN
    ;OF SUM
    ;
        FFOA            0
        FSTA            SMSUM
        FSTA            SMXXSUM
        FSTA            XCNT
    ;
    LOOP
        LHLD            OFFSET
        LXI             D, RCDATA
        DAD             D
        MOV             E, M
        INX             H
        MOV             D, M
        SDED            IRCV
    ;
        LHLD            OFFSET
        LXI             D, ABDATA
        DAD             D
        MOV             E, M
        INX             H
        MOV             D, M
        SDED            IABV
    ;
```

```
        CALL            SUMMERS
    ;
        LHLD            OFFSET
        INX             H
        INX             H
        SHLD            OFFSET
        LXI         .   D, 1000
        CALL            TDHE
        IF (PSW, IS, ZERO)
          LXI           H, 0
          SHLD          OFFSET
        ENDIF
    ;

    ;
    ;QUIT WHEN OFFSET = DLTOFF
        LHLD            OFFSET
        LDED            DLTOFF  .
        CALL            TDHE
        IF (PSW,. IS, ZERO)
          RET           ;EXIT LOOP
        ENDIF
    ENDLOOP
    ;COMPUTE MEAN AND STD OF SUM
        FLDB            SMSUM
        FLDA            XCNT
        FDIV
        FSTA            SMMEAN
    ;
        FLDB            SMSUM
        FLDA            SMSUM
        FMUL
        FATB
        FLDA            XCNT
        FDIV
        FLDB            SMXXSUM
        FSUB
        FATB
        FLDA            XCNT
        FDIV
        FSQR
        FSTA            SMSTD
    ;HIGH LIMIT = SMMEAN + QDCSTD*SMSTD
    ;LOW LIMIT = SMMEAN − QDCSTD*SMSTD
    ;
        FLDB            QDCSTD
        FLDA            SMSTD
        FMUL
        FLDB            SMMEAN
        FADD
        FSTA            SMHIGH
    ;
        FLDB            QDCSTD
```

```
        FLDA            SMSTD
        FMUL
        FLDB            SMMEAN
        FSUB
        FSTA            SMLOW
     ;
     ;NOW COMPUTE MEAN AND STD OF RC AND AB FOR ALL PAIRS WHERE
     ;RC+AB < SMHIGH AND RC+AB > SMLOW
     ;
     ;
        LDA             DLTFULL
        IF ( .A, IS, ZERO)      ;RING NOT FULL YET
          LXI           H, 0
          SHLD          OFFSET
        ELSE                    ;RING IS FULL
          LHLD          DLTOFF  ;POINTS TO NEXT PLACE TO PUT, SO WILL BE
                                ;THE OLDEST VALUE
          SHLD          OFFSET
        ENDIF
     ;     .
     ;
        FFOA            0
        FSTA            RCSUM
        FSTA            RCXXSUM
        FSTA            ABSUM
        FSTA            ABXXSUM
        FSTA            XCNT


     ;
LOOP
        LHLD            OFFSET
        LXI             D, RCDATA
        DAD             D
        MOV             E, M
        INX             H
        MOV             D, M
        SDED            IRCV
     ;
        LHLD            OFFSET
        LXI             D, ABDATA
        DAD             D
        MOV             E, M
        INX             H
        MOV             D, M
        SDED            IABV
     ;
        CALL            RASUMMERS
     ;
        LHLD            OFFSET
        INX             H
        INX             H
        SHLD            OFFSET
        LXI             D, 1000
```

```
        CALL            TDHE
        IF (PSW, IS, ZERO)
          LXI           H, 0
          SHLD          OFFSET
        ENDIF
   ;
   ;
   ;QUIT WHEN OFFSET = DLTOFF
        LHLD            OFFSET
        LDED            DLTOFF
        CALL            TDHE
        IF (PSW, IS, ZERO)
          RET           ;EXIT LOOP
        ENDIF
   ENDLOOP
   ;
   ;IF XCNT < 2, THEN NO GOOD DATA
        FLDB            XCNT
        FFOA            2
        FSUB      .
        FTST            AREG, LT
        IF (PSW, IS, ZERO)
          LXI           H, NODELTS
          CALL          TXTYP
          STC
          RET
        ENDIF
   ;COMPUTE MEAN OF RC AND AB
        FLOB            RCSUM
        FLDA            XCNT
        FDIV
        FSTA            FRC
   ;
        FLDB            ABSUM
        FLDA            XCNT
        FDIV
        FSTA            FAB
   ;
   ;COMPUTE STD OF RC AND STD OF AB
   ;
        FLDB            RCSUM
        FLDA            RCSUM
        FMUL
        FATB
        FLDA            XCNT
        FDIV
        FLDB            RCXXSUM
        FSUB
        FATB
        FLDA            XCNT
        FDIV
        FSQR
```

```
        FSTA              RCSTD
    ;
        FLDB              ABSUM
        FLDA              ABSUM
        FMUL
        FATB
        FLDA              XCNT
        FDIV
        FLDB              ABXXSUM
        FSUB
        FATB
        FLDA              XCNT
        FDIV
        FSQR
        FSTA              ABSTD
    ;MAKE VALUES ML
        FLDB              FRC
        FLDA              CCS
        FMUL
        FSTA              FRC
    ;
        FLDB              FAB
        FLDA              CCS
        FMUL
        FSTA              FAB
    ;
        FLDB              ABSTD
        FLDA              CCS
        FMUL
        FSTA              ABSTD
    ;
        FLDB              RCSTD
        FLDA              CCS
        FMUL
        FSTA              RCSTD
    ;
    ;COV = STD/MEAN
        FLDB              RCSTD
        FLDA              FRC
        FDIV
        FATB
        FFOA              100
        FMUL
```

```
       FSTA            COVRC
;
       FLDB            ABSTD
       FLDA            FAB
       FDIV
       FATB
       FFOA            100
       FMUL
       FSTA            COVAB
       ORA             A
       RET
;
;
;
RASUMMERS:
       FILA            IRCV
       FLOT
       FSTA            FRC

       FILA            IABV
       FLOT
       FSTA            FAB
;
;DO NOT USE IF CRC+FAB > SMHIGH OR IF FRC+FAB < SMLOW
       FLDB            FRC
       FLDA            FAB
       FADD
       FATB
       FLDA            SMHIGH
       FSUB
       FTST            AREG, GT
       IF (PSW, IS, ZERO)
          RET
       ENDIF
;
       FLDB            FRC
       FLDA            FAB
       FADD
       FATB
       FLDA            SMLOW
       FSUB
       FTST            AREG, LT
       IF (PSW, IS, ZERO)
          RET
       ENDIF
;
;
;
       FLDA            FRC
       FLDB            RCSUM
       FADD
       FSTA            RCSUM
;
       FLDB            FRC
       FLDA            FRC
       FMUL
       FLDB            RCXXSUM
       FADD
       FSTA            RCXXSUM
;
       FLDA            FAB
```

```
        FLDB            ABSUM
        FADD
        FSTA            ABSUM
    ;
        FLDB            FAB
        FLDA            FAB
        FMUL
        FLDB            ABXXSUM
        FADD
        FSTA            ABXXSUM

    ;
    ;
    ;
        FLDB            XCNT
        FFOA            1
        FADD
        FSTA            XCNT
    ;
        RET

    ;
    ;
    ;
    ;
    SUMMERS:
        FILA            IRCV
        FLOT
        FATB
        FILA            IABV
        FLOT
        FADD
        FSTA            FSUM

        FLDA            FSUM
        FLDB            SMSUM
        FADD
        FSTA            SMSUM
    ;
        FLDA            FSUM
        FLDB            FSUM
        FMUL
        FLDB            SMXXSUM
        FADD
        FSTA            SMXXSUM
    ;
    ;
        FLDB            XCNT
        FFOA            1
        FADD
        FSTA            XCNT
        RET
    ;
    ;
    ;
    ;
        . END
```

FILE: CFIND.ASM

```
;
        .IDENT          CFIND
        .INSERT         SP80.ASM
        .INSERT         CALCOM
        .INSERT         SELCOM
        .INSERT         EFFCOM
        .INSERT         DRVCOM
        .INSERT         SIOCOM
        .INSERT         FPMAC.SRC
        .INSERT         PKCOM.ASM
        .ENTRY          FIND, GETP2, DERIV5
        .EXTERN         TDHE, PTRUPD
        FINIT
FIND:
;
;HL = OFFSET OF CURRENT PT ON ENTRANCE
;
;ON EXIT: IF CP FOUND, THEN CARRY SET AND
;     HL =·         SPIROMETER PT
;     DE =          RIB CAGE PT
;     BC =          ABDOMEN PT
        SHLD            CUROFF
;
        LHLD            CUROFF
        LXI             D, SPBUFF
        DAD             D
        MOV             E, M
        INX             H
        MOV             D, M
        SDED            CURSP
;
        LHLD            CUROFF
        LXI             D, RCBUFF
        DAD             D
        MOV             E, M
        INX             H
        MOV             D, M
        SDED            CURRC
;
        LHLD            CUROFF
        LXI             D, ABBUFF
        DAD             D
        MOV             E, M
        INX             H
        MOV             D, M
        SDED            CURAB
;
        LHLD            CUROFF
        LXI             D, TMBUFF
        DAD             D
        MOV             E, M
        INX             H
        MOV             D, M
```

```
          SDED            CURTM
     ;
     ;
          CALL            GTSIGN      ;CHECK SIGNS OF DERIVATIVES
     ;
     ;
          LDA             PTF
          IF (.A, IS, NZERO)     ;LOOK FOR MIN
            LHLD          SPTEMP
            LDED          CURSP
            ORA           A
            DSBC          D
          IF (PSW, IS, NCARRY)       ;NEW TMP MIN FOUND
            LHLD          CURSP
            SHLD          SPTEMP
            LHLD          CURRC
            SHLD          RCTEMP
            LHLD          CURAB
            SHLD          ABTEMP
            LHLD          CUROFF
            SHLD          OFFTEMP
            LHLD          CURTM
            SHLD          TMTEMP

;1. ADD CURRENT RCDERV PT TO RC COUNTS
;2. ADD RCTNT TO RC COUNTS
;3. RCTCNT<== 0
;4. ADD CURRENT ABDERV TO AB COUNTS
;5. ADD ABTCNT TO AB COUNTS
;6. ABTCNT <== 0
                CALL    UPDT1

            ORA           A
            RET
          ELSE                  ;POSSIBLE CP SO CHECK SLOPE BETWEEN
                                ;CURRNT PT AND TEMP PT
            LHLD          CURSP
            LDED          SPTEMP
            ORA           A
            DSBC          D
            ;NOW HL HOLDS DIFFERENCE
            LDED          MINCC
            ORA           A
            DSBC          D
          IF (PSW, IS, NCARRY)       ;THEN SPTEMP IS CP

                LHLD      OFFTEMP
                SHLD      CPOFF
                LHLD      SPTEMP
                PUSH      H
                LHLD      RCTEMP
                PUSH      H
                LHLD      ABTEMP
                PUSH      H
```

```
            LHLD      CURSP
            SHLD      SPTEMP
            LHLD      CURRC
            SHLD      RCTEMP
            LHLD      CURAB
            SHLD      ABTEMP
            LHLD      CUROFF
            SHLD      OFFTEMP
            LHLD      TMTEMP
            SHLD      CPTIME       ;TIME OF THIS CP
    ;
            LHLD      CURTM
            SHLD      TMTEMP        ;NEW TEMP
    ;
    ;1. STORE RC COUNTS
    ;2. RC COUNTS <== RCTCNT
    ;3. RCTCNT <== 0
    ;4. STORE AB COUNTS
    ;5. AB COUNTS <== ABTCNT
    ;6. ABTCNT <== 0
    ;
            CALL      UPDT2
    ;
            POP       B       ;AB VALUE
            POP       D       ;RC VALUE
            POP       H       ;SP VALUE
            STC         ;SET CARRY
            RET
          ELSE
    ;NOT A LARGE ENOUGH SLOPE
    ;
    ;1 ADD RCDERV TO RCTCNT
    ;2. ADD ABDERV TO ABTCNT
            CALL      UPDT3
            ORA       A
            RET
        ENDIF
      ENDIF
    ELSE                  ;LOOK FOR MAX
      LHLD   SPTEMP
        LDED   CURSP
      XCHG
      ORA    A
      DSBC   D
      IF (PSW, IS, NCARRY)        ;;THEN CURRENT >= SPTEMP
            LHLD      CURSP
            SHLD      SPTEMP
            LHLD      CURRC
            SHLD      RCTEMP
            LHLD      CURAB
            SHLD      ABTEMP
            LHLD      CUROFF
            SHLD      OFFTEMP
```

```
        LHLD        CURTM
        SHLD        TMTEMP
  ;
            CALL        UPDT1
        ORA A
        RET
      ELSE        ;POSSIBLE CP
        LHLD        SPTEMP
        LDED        CURSP
        ORA A
        DSBC        D
  ;NOW HL = DIFF
        LDED        MINCC
        ORA A
        DSBC        D
      IF (PSW, IS, NCARRY)        ;THEN SPTEMP IS CP
            LHLD    OFFTEMP
            BHLD    CPOFF
            LHLD    BPTEMP
        .   PUSH    H
            LHLD    RCTEMP
            PUSH    H
            LHLD    HBTEMP
            PUSH    H
  ;
            LHLD    TMTEMP
            SHLD    OFTIME        ;TIME OF CP
  ;
            LHLD    CURSP
            SHLD    GPTEMP
            LHLD    CURRC
            SHLD    RCTEMP
            LHLD    CURAB
            SHLD    ABTEMP
            LHLD    CUROFF
            SHLD    OFFTEMP
            LHLD    CURTM
            SHLD    TMTEMP
  ;
            CALL    UPDT2
            POP     B       ;AB VALUE
            POP     D       ;RC VALUE
            POP     H       ;SP VALUE
            STC
            RET
      ELSE        ;NOT LARGE ENOUGH SLOPE
            CALL    UPDT3
            ORA     A
              RET
      ENDIF
    ENDIF
  ENDIF
```

```
;
;
;
;
;GETP2:
;
;SEE IF AT LEAST 5 POINTS IN RING BUFFER
;
;    LHLD    RIPT
;    LXI     D.10
     ORA     A
     DSBC    D
     IF(PSW,IS,CARRY)         ;THEN < 5 PTS
       STC
     ELSE
       ORA   A
     ENDIF
     RET
;
;
UPDT1:
;
;1. ADD CURRENT ABS(RCDERV) PT TO RC COUNTS
;2. ADD RCTCNT TO RC COUNTS
;3. RCTCNT <== 0
;4. ADD CURRENT ABS(ABDERV) TO AB COUNTS
;5. ADD ABTCNT TO AB COUNTS
;6. ABTCNT <== 0
;
;
;
;******
;NOTE: DO NOT SEPARATE INPHASE AND OUT "OF PHASE FIR AND"
;*****
             LHLD    ABSRCD
             LDED    RCIN
             DAD     D
             SHLD    RCIN
;
;
             LHLD    RCIN
             LDED    RCTICNT
             DAD     D
             SHLD    RCIN
;
;
             LXI     H, 0
             SHLD    RCTICNT
;
;
             LHLD    ABSABD
             LDED    ABIN
             DAD     D
             SHLD    ABIN
;
;
             LHLD    ABIN
             LDED    ABTICNT
             DAD     D
```

```
        SHLD    ABIN
;

        LXI     H, 0
        SHLD    ABTICNT
;
;SUM: DO NOT SEPARATE INPHASE AND OUT OF PHASE VALUES
;    ADD THEM ALL
        LHLD    ABSSMD
        LDED    SMIO
        DAD     D
        SHLD    SMIO
;
;

        LHLD    SMIO
        LDED    SMTCNT
        DAD     D
        SHLD    SMTCNT
;
;
        .   LXI     H, 0
        SHLD    SMTCNT
;
;FOR PEAK FLOW STUFF:
;SMDERV = CURRENT FLOW, ABSSMD = ABS(SMDERV)
;
;IF ON INSP SIDE:, LOOKING FOR MAX (PTF=0)
   LDA      PTF
   IF (.A, IS, ZERO       ;ON INSP SIDE
;IF CURRENT FLOW > 0, OR IF ABSSMD = SMDERV
      LHLD          ABSSMD
      LDED          SMDERV
      CALL          TDHE
      IF (PSW, IS, ZERO)       ;THEN CURRENT FLOW > 0
;IF CURRENT FLOW > TEMPK
           LHLD    TEMPK
           LDED    SMDERV
           ORA     A
           DSBC    D
           IF (PSW, IS, CARRY)        ;CURRENT FLOW > TEMPK
              LHLD        SMDERV
              SHLD        TEMPK
              LHLD        CURTM
              SHLD        PKTIME
        ENDIF
     ENDIF
;IF TTPOS > TEMPK
```

```
        LHLD        TEMPK
        LDED        TTPOS
        OAR A
        DSBC        D
        IF (PSW, IS, CARRY)      ;TTPOS > TEMPK
            LHLD    TTPOS
            SHLD    TEMPK
            LHLD    TTPTM
            SHLD    PKTIME
        ENDIF
;
        LXI, H, 0
        SHLD        TTPOS
        SHLD        TTNEG
        LHLD        CURTM
        SHLD        TTPTM
        SHLD        TTNTM
;
    ELSE                ;ON THE EXP SIDE <LOOKING FOR MIN
;
;IF CURRENT FLOW < 0, OR IF ABSSMD NOT= SMDERV
            LHLD        ABSSMD
            LDED        SMDERV
            CALL        TDHE
        IF (PSW, IS, NZERO)       ;THEN CURRENT FLOW 0
;IF ABS(CURRENT FLOW) > TEMPK
            LHLD    TEMPK
            LDED    ABSSMD
            ORA     A
            DSBC    D
            IF (PSW, IS, CARRY)       ;ABS(CURRENT FLOW) > TEMPK
                LHLD        ABSSMD
                SHLD        TEMPK
                LHLD        CURTM
                SHLD        PKTIME
            ENDIF
        ENDIF
;IF TTNEG > TEMPK
        LHLD        TEMPK
        LDED        TTNEG
        ORA A
        DSBC        D
        IF (PSW, IS CARRY)       ;TTNEG > TEMPK
            LHLD    TTNEG
            SHLD    TEMPK
            LHLD    TTNTM
            SHLD    PKTIME
        ENDIF
;
        LXI H, 0
        SHLD        TTPOS
        SHLD        TTNEG
        LHLD        CURTM
```

66

```
        SHLD          TTPTM
        SHLD          TTNTM
;
;
   ENDIF


                      RET
;
;
UPDT2:
;
;1. STORE RC COUNTS
;2. RC COUNTS <== RCTCNT
;3. RCTCNT <== 0
;4. STORE AB COUNTS
;5. AB COUNTS <== ABTCNT
;6. ABTCNT <== 0
        LHLD          RCIN
        SHLD          RCINVOL
;
;
        LHLD          RCTICNT
        SHLD          RCIN
;
;
        LXI           H, 0
        SHLD          RCTICNT
;
        LHLD          ABIN
        SHLD          ABINVOL
;
;
        LHLD          ABTICNT
        SHLD          ABIN
;
;
        LXI           H, 0
        SHLD          ABTICNT
;
;SUM: DO NOT SEPARATE INPHASE AND OUT OF PHASE
        LHLD          SMIO
        SHLD          SMVOL
;
;
        LHLD          SMTCNT
        SHLD          SMIO
;
;
        LXI           H, 0
        SHLD          SMTCNT
;
;FOR PEAK FLOW STUFF:
;SMDERV = CURRENT FLOW,        ABSSMD = ABS(SMDERV)
```

```
;
;IF ON INSP SIDE:, LOOKING FOR MAX (PTF=0)
      LDA              PTF
      IF (.A, IS, ZERO)       ;ON INSP SIDE
;TEMPK IS THE PEAK FLOW, AND PKTIME IS THE TIME OF IT
;STORE THE TIME OF THE PEAK FLOW
      CALL             PKSTORE
      LHLD             TTNEG       ;NOW WILL BE ON EXP SIDE
      SHLD             TEMPK
      LHLD             TTNTM
      SHLD             PKTIME
      LXI              H, 0
      SHLD             TTPOS
      SHLD             TTNEG
      LHLD             CURTM
      SHLD             TTPTM
      SHLD             TTNTM
      ELSE                      :ON EXP SIDE
;TEMPK IS THE PEAK FLOW, AND PKTIME IS THE TIME OF IT
;STORE THE TIME OF THE PEAK FLOW
      CALL             PKSTORE
      LHLD             TTPOS       ;NOW WILL BE ON INSP SIDE
      SHLD             TEMPK
      LHLD             TTPTM
      SHLD             PKTIME
      LXI              H, 0
      SHLD             TTPOS
      SHLD             TTNEG
      LHLD             CURTM
      SHLD             TTPTM
      SHLD             TTNTM
      ENDIF
;
      RET
;
;
;
;
UPDT3:
;
;1. ADD ABS(RCDERV) TO RCTCNT
;2. ADD ABS(ABDERV) TO ABTCNT
;
      LHLD             ABSRCD
      LDED             RCTICNT
      DAD              D
      SHLD             RCTICNT
;
      LHLD             ABSABD
      LDED             ABTICNT
      DAD              D
      SHLD             ABTICNT
```

```
;
;SUM: DO NOT SEPARATE INPHASE AND OUT OF PHASE
;
        LHLD            ABSSMD
        LDED            SMTCNT
        DAD             D
        SHLD            SMTCNT
;
;
;FOR PEAK FLOW STUFF:
;SMDERV = CURRENT FLOW, ABSSMD = ABS(SMDERV)
;
;IF CURRENT FLOW > 0
        LHLD            SMDERV
        LDED            ABSSMD
        CALL            TDHE
        IF (PSW, IS, ZERO)      ;THEN CURRENT FLOW > 0
;IF CURRENT FLOW > TTPOS
        LHLD            TTPOS
        LDED            SMDERV
        ORA             A
        DSBC            D
        IF (PSW, IS, CARRY)
                        LHLD            SMDERV
                        SHLD            TTPOS
                        LHLD            CURTM
                        SHLD            TTPTM
        ENDIF
    ELSE
;IF ABS(CURRENT FLOW) > TTNEG
        LHLD            TTNEG
        LDED            ABSSMD
        ORA             A
        DSBC            D
        IF (PSW, IS, CARRY)
                        LHLD            ABSSMD
                        SHLD            TTNEG
                        LHLD            CURTM
                        SHLD            TTNTM
        ENDIF
    ENDIF
;
        RET
;
;
;
PKSTORE:
        LHLD            PKTIME
        SHLD            PKVALUE
        RET
;
;
```

```
;
GTSIGN:
    LHLD            RCDERV          ;CURRENT RC DERIV VALUE
    MOV             A, H
    ANI             80H
    STA             RCSIGN
;
    LHLD            ABDERV          ;CURRENT AB DERIV VALUE
    MOV             A, H
    ANI             80H
    STA             ABSIGN
;
    LHLD            SMDERV
    MOV             A, H
    ANI             80H
    STA             SMSIGN
;
;
    MOV             B, A
    LDA             RCSIGN
    IF (.A, EQ, .B)     ;THEN RC INPHASE W/ SUM
        XRA         A
        CMA
        STA         RCINPHS
    ELSE
        XRA         A
        STA         RCINPHS
    ENDIF
;
    LDA             SMSIGN
    MOV             B, A
    LDA             ABSIGN
    IF (.A, EQ, .B)     ;THEN AB INPHASE W/ SUM
        XRA         A
        CMA
        STA         ABINPHS
    ELSE
        XRA         A
        STA         ABINPHS
    ENDIF
    RET
;
;
;
;
;
;
;
;
;
;
DERIV5:
;*********************
;DERIV USING 9 POINTS
```

# EP 0 256 115 B1

```
;**********************
;
;DERIVATIVE USING 5 POINTS
;
;SRCOFST HOLDS OFFSET TO GET Y5 FROM RAW DATA BUFFERS
;
;ASSUME: RCY1, RCY2, RCY3, RCY4, ABY1, ABY2, ABY3, ABY4 ALREADY SET
;
    LXI         D, RCBUFF
    LHLD        SRCOFST
    DAD         D
    MOV         E, M
    INX         H
    MOV         D, M
    SDED        RCY9
;
    LHLD        SRCOFST
    LXI         D, ABBUFF
    DAD         D
    MOV·        E, M
    INX         H
    MOV         D, M
    SDED        ABY9
;
    LXI         B, RSIZE        ;UPDATE FOR NEXT TIME
    LHLD        SRCOFST
    CALL        PTRUPD
    SHLD        SRCOFST
;DERIV = (9—1)*4 + (8—2)*3 + (7—3)*2 + (6—4)
;
    LHLD        RCY9
    LDED        RCY1
    ORA         A
    DSBC        D
    DAD         H       ;(RCY9—RCY1)*2
    DAD         H       ;(RCY9—RCY1)*4
    PUSH        H
;
    LHLD        RCY8
    LDED        RCY2
    ORA         A
    DSBC        D
    MOV         E, L
    MOV         D, H
    DAD         H       ;*2
    DAD         D       ;*3
    POP         D
    DAD         D
    PUSH        H
;
    LHLD        RCY7
    LDED        RCY3
    ORA         A
```

71

```
      DSBC            D
      DAD             H
      POP             D
      DAD             D
      PUSH            H
;
      LHLD            RCY6
      LDED            RCY4
      ORA             A
      DSBC            D
      POP             D
      DAD             D
      SHLD            RCDERV
;
;*****: DIVIDE RCDERV BY 4 TO AVOID OVERFLOW WHEN INTEGRATING IT
      LHLD            RCDERV
      SRAR            H
      RARR            L
      SRAR            H
      RARR     .      L
      SHLD            RCDERV

;***
;DERIV = (9—1)*4 + (8—2)*3 + (7—3)*2 + (6—4)
;
      LHLD            ABY9
      LDED            ABY1
      ORA             A
      DSBC            D
      DAD             H       ;(ABY9—ABY1)*2
      DAD             H       ;(ABY9—ABY1)*4
      PUSH            H
;
      LHLD            ABY8
      LDED            ABY2
      ORA             A
      DSBC            D
      MOV             E, L
      MOV             D, H
      DAD             H       ;*2
      DAD             D       ;*3
      POP             D
      DAD             D
      PUSH            H
```

```
;
    LHLD            ABY7
    LDED            ABY3
    ORA             A
    DSBC            D
    DAD             H
    POP             D
    DAD             D
    PUSH            H
;
    LHLD            ABY6
    LDED            ABY4
    ORA             A
    DSBC            D
    POP             D
    DAD             D
    SHLD            ABDERV
;
;*****: DIVIDE ABDERV BY 4 TO AVOID OVERFLOW WHEN INTEGRATING IT
    LHLD·           ABDERV
    BRAR            H
    RARR            L
    SRAR            H
    RARR            L
    SHLD            ABDERV

;***
;
;
;RESET VALUES FOR NEXT TIME
    LHLD            RCY2
    SHLD            RCY1
    LHLD            RCY3
    SHLD            RCY2
    LHLD            RCY4
    SHLD            RCY3
    LHLD            RCY5
    SHLD            RCY4
    LHLD            RCY6
    SHLD            RCY5
    LHLD            RCY7
    SHLD            RCY6
    LHLD            RCY8
    SHLD            RCY7
    LHLD            RCY9
    SHLD            RCY8
;
    LHLD            ABY2
    SHLD            ABY1
    LHLD            ABY3
    SHLD            ABY2
    LHLD            ABY4
    SHLD            ABY3
    LHLD            ABY5
    SHLD            ABY4
    LHLD            ABY6
    SHLD            ABY5
    LHLD            ABY7
    SHLD            ABY6
    LHLD            ABY8
    SHLD            ABY7
    LHLD            ABY9
    SHLD            ABY8
```

```
           ;
           ;
           ;
           LHLD             RCDERV
           LDED             ABDERV
           DAD              D
           SHLD             SMDERV
           ;
           ;
           ;
           ;COMPUTE ABSOLUTE VALUE OF RCDERV AND ABDERV, AND SMDERV
           LHLD             RCDERV
           MOV              A, H
           ANI              80H
           IF (.A, IS, NZERO)       ;THEN RCDERV < 0
             MOV            A, L
             CMA
             MOV            L, A
             MOV            A, H
             CMA
             MOV            H, A
             INX            H
             SHLD           ABSRCD
           ELSE
             SHLD           ABSRCD
           ENDIF
           ;
           LHLD             ABDERV
           MOV              A, H
           ANI              80H
           IF (.A, IS, NZERO)       ;THEN ABDERV < 0
             MOV            A, L
             CMA
             MOV            L, A
             MOV            A, H
             CMA
             MOV            H, A
             INX            H
             SHLD           ABSABD
           ELSE
             SHLD           ABSABD
           ENDIF
           ;
           LHLD             SMDERV
           MOV              A, H
           ANI              80H
           IF (.A, IS, NZERO)       ;THEN SMDERV < 0
             MOV            A, L
             CMA
             MOV            L, A
             MOV            A, H
             CMA
             MOV            H, A
             INX            H
             SHLD           ABSSMD
```

74

```
          ELSE
            SHLD          ABSSMD
          ENDIF
        ;
        ;
        ;
          RET
        ;
        ;
        ;
        ;
        ;
        ;
        ;
GETP2;
        ;
        ;SEE IF AT LEAST 10 POINTS IN RING BUFFER
        ;
          LHLD          RIPT
          LXI  ·        D, 20
          ORA           A
          DSBC          D
          IF (PSW, IS, CARRY)      ;THEN < 10 PTS
            STC
          ELSE
        ;
        ;
        ;SET UP INIT VALUES IN RCY1, RCY2, RCY3, RCY4
        ;       ABY1, ABY2, ABY3, ABY4, and SRCOFST
        ;
          LXI           H, RCBUFF
          MOV           E, M
          INX           H
          MOV           D, M
          SDED          RCY1
        ;
          INX           H
          MOV           E, M
          INX           H
          MOV           D, M
          SDED          RCY2
        ;
          INX           H
          MOV           E, M
          INX           H
          MOV           D, M
          SDED          RCY3
        ;
          INX           H
          MOV           E, M
          INX           H
          MOV           D, M
          SDED          RCY4
```

75

```
;
        INX         H
        MOV         E, M
        INX         H
        MOV         D, M
        SDED        RCY5
;
        INX         H
        MOV         E, M
        INX         H
        MOV         D, M
        SDED        RCY6
;
        INX         H
        MOV         E, M
        INX         H
        MOV         D, M
        SDED        RCY7
;
        INX         H
        MOV         E, M
        INX         H
        MOV         D, M
        SDED        RCY8
;
;
        LXI         H, ABBUFF
        MOV         E, M
        INX         H
        MOV         D, M
        SDED        ABY1
;
        INX         H
        MOV         E, M
        INX         H
        MOV         D, M
        SDED        ABY2
;
        INX         H
        MOV         E, M
        INX         H
        MOV         D, M
        SDED        ABY3
;
        INX         H
        MOV         E, M
        INX         H
        MOV         D, M
        SDED        ABY4
;
        INX         H
        MOV         E, M
        INX         H
```

```
        MOV             D, M
        SDED            ABY5
    ;
        INX             H
        MOV             E, M
        INX             H
        MOV             D, M
        SDED            ABY6
    ;
        INX             H
        MOV             E, M
        INX             H
        MOV             D, M
        SDED            ABY7
    ;
        INX             H
        MOV             E, M
        INX             H
        MOV             D, M
        SDED ·          ABY8
    ;
        LXI             H,14        ;OFFSET FOR THE 9TH VALUE IN BUFFERS
        SHLD            SCROFST
    ;
        LXI             H,8         ;OFFSET OF MIDDLE OF DERIV
        SHLD            ROPT
    ;
        LXI             H, 0
        SHLD            INTGRC
        ORA             A
        ENDIF
        RET
    ;
    ;
        .EXTERN         CFD
        .LOC            CFD
    CUROFF:             .BLKB       2
    OFFTEMP:            .BLKB       2
        .RELOC
    ;
    ;
        .END
    ;**************************************.
    ;
    ;
    ;
        .IDENT          DKFIND
        .INSERT         SP80.ASM
        .INSERT         CALCOM
        .INSERT         FPMAC.SRC
        .INSERT         EFFCOM
        .INSERT         DKCOM
        .INSERT         DRVCOM
        .INSERT         SIOCOM
```

```
        .ENTRY          DKFIND, DKGETP2
        .EXTERN         TDHE
        .ENTRY          DERIV5
        .EXTERN         PTRUPD
        FINIT
;
;
;ASSUME: THAT ALL RC VALUES, AB VALUES AND SUM VALUES
;        WILL HAVE FIRST 4 BITS = 000H COMING FROM
;        A—D.
;
OKFIND:
;
;HL = OFFSET OF CURRENT PT ON ENTRANCE
;
;ON EXIT: IF CP FOUND, THEN CARRY SET AND
;   HL = SUM PT
;   DE = RIB CAGE PT
;   BC = ABDOMEN PT
:     TVALUE = TIME OF CP
;
        PUSH            H
        PUSH            H
        PUSH            H
        LXI             D, SPBUFF
        DAD             D
        MOV             E, M
        INX             H
        MOV             D, M
        SDED            CURSP
;
        POP             H
        LXI             D, RCBUFF
        DAD             D
        MOV             E, M
        INX             H
        MOV             D, M
        SDED            CURRC
;
        POP             H
        LXI             D, ABBUFF
        DAD             D
        MOV             E, M
        INX             H
        MOV             D, M
        SDED            CURAB
;
        POP             H
        LXI             D, TMBUFF
        DAD             D
        MOV             E, M
        INX             H
        MOV             D, M
```

78

```
        SDED            CURTM
        ;
        ;
        ;
        ;
        ;
        ;
        ;
        CALL            GTSIGN          ;CHECK SIGNS OF DERIVATIVES
        ;
        ;
        LDA             PTF
        IF (.A, IS, ZERO)       ;LOOK FOR MIN
            LHLD        SPTEMP
            LDED        CURSP
            ORA         A
            DSBC        D
            IF (PSW, IS, CARRY)         ;NEW TMP MIN FOUND

                LHLD        CURSP
                SHLD        SPTEMP
                LHLD        CURRC
                SHLD        RCTEMP
                LHLD        CURAB
                SHLD        ASTEMP
                LHLD        CURTM
                SHLD        SMTIME
        ;
        ;1. ADD CURRENT RCDERV 8T TO RC COUNTS
        ;2. ADD RCTCNT TO RC COUNTS
        ;3. RCTCNT <==0
        ;4. ADD CURRENT ABDERV TO AB COUNTS
        ;5. ADD ABTCNT TO AB COUNTS
        ;6. ABTCNT <==0
                CALL        UPDT1

        ;
        ;
        ;
                ORA         A
                RET
        ELSE                    ;POSSIBLE CP SO CHECK SLOPE BETWEEN
                                ;CURRENT PT AND TEMP PT
                LHLD        CURSP
                LDED        SPTEMP
                ORA         A
                DSBC        D
                ;NOW HL HOLDS DIFFERENCE
                LDED        MINCC
                ORA         A
                DSBC        D
                IF (PSW, IS, NCARRY)        ;THEN SMTEMP IS CP
                    LHLD        SPTEMP
                    PUSH        H
                    LHLD        RCTEMP
```

79

```
        PUSH    H
        LHLD    ABTEMP
        PUSH    H
        LHLD    SMTIME
        SHLD    TVALUE
        LHLD    CURSP
        SHLD    SPTEMP
        LHLD    CURRC
        SHLD    RCTEMP
        LHLD    CURAB
        SHLD    ABTEMP
        LHLD    CURTM
        SHLD    SMTIME
;
;1. STORE RC COUNTS
;2. RC COUNTS <==RCTCNT
;3. RCTCNT <==0
;4. STORE AB COUNTS
;5. AB COUNTS <== ABTCNT
;6. ABTCNT <==0
;
        CALL    UPDT2
;
;
        POP     B       ;AB VALUE
        POP     D       ;RC VALUE
;
;SET THE APPROPRIATE BIT ON RC VALUE TO INDICATE
;A MINIMUM BRTH
        MOV     A, D
        ANI     0FH         ;FIRST 4 BITS SHOULD = 00009
        MOV     D, A
;
        POP     H       ;SM VALUE
        STC         ;SET CARRY
        RET
    ELSE
    ;NOT A LARGE ENOUGH SLOPE
;
;1. ADD RCDERV TO RCTCNT
;2. ADD ABDERV TO ABTCNT
        CALL    UPDT3
;
        ORA     A
        RET
    ENDIF
  ENDIF
 ELSE                   ;LOOK FOR MAX
    LHLD  SPTEMP
     LDED  CURSP
    XCHG
    ORA  A
    DSBC  D
```

```
        IF (PSW, IS, NCARRY)        ;;THEN CURRENT >= SMTEMP
            LHLD        CURSP
            SHLD        SPTEMP
            LHLD        CURRC
            SHLD        RCTEMP
            LHLD        CURAB
            SHLD        ABTEMP
                LHLD        CURTM
                SHLD        SMTIME
;
                CALL        UPDT1
    ;
            ORA   A
            RET
        ELSE   ;POSSIBLE CP
            LHLD        SPTEMP
            LDED        CURSP
            ORA   A
            DSBC        D
;NOW HL = DIFF
            LDED        MINCC
            ORA   A
            DSBC        D
            IF (PSW, IS, NCARRY)        ;THEN SMTEMP IS CP
                LHLD        SPTEMP
                PUSH        H
                LHLD        RCTEMP
                PUSH        H
                LHLD        ABTEMP
                PUSH        H
                LHLD        SMTIME
                SHLD        TVALUE
                LHLD        CURSP
                SHLD        SPTEMP
                LHLD        CURRC
                SHLD        RCTEMP
                LHLD        CURAB
                SHLD        ABTEMP
                LHLD        CURTM
                SHLD        SMTIME
    ;
    ;
                CALL        UPDT2
                POP        B        ;AB VALUE
                POP        D        ;RC VALUE
    ;
        ;SET THE APPROPRIATE BIT ON RC VALUE TO INDICATE
        ;A MAXIMUM BRTH
                MOV        A, D
                ORI        10H        ;FIRST 4 BITS SHOULD = 00018
                MOV        D, A
    ;
                POP        H        ;SM VALUE
```

81

```
            STC
            RET
        ELSE            ;NOT LARGE ENOUGH SLOPE
            CALL    UPDT3
            ORA     A
            RET
        ENDIF
      ENDIF
    ENDIF
;
;
;
    UPDT1:

;1. ADD CURRENT ABS(RCDERV) PT TO RC COUNTS
;2. ADD RCTCNT TO RC COUNTS
;3. RCTCNT <==0
;4. ADD CURRENT ABS(ABDERV) TO AB COUNTS
;5. ADD ABTCNT TO AB COUNTS
;6. ABTCNT <==0
;
            LHLD    ABSRCD
            LDA     RCINPHS
            IF (.A, IS, NZERO)       ;THEN RC INPHASE WITH SUM
              LDED    RCIN
              DAD     D
              SHLD    RCIN
;
            ELSE                     ;RC OUT OF PHASE WITH SUM
              LDED    RCOUT
              DAD     D
              SHLD    RCOUT
;
            ENDIF
;
            LHLD    RCIN
            LDED    RCTICNT
            DAD     D
            SHLD    RCIN
;
            LHLD    RCOUT
            LDED    RCTOCNT
            DAD     D
            SHLD    RCOUT
;
            LXI     H, 0
            SHLD    RCTICNT
            SHLD    RCTOCNT
;
;
            LHLD    ABSABD
            LDA     ABINPHS
            IF (.A, IS, NZERO)       ;THEN AB INPHASE WITH SUM
```

82

```
            LDED    ABIN
            DAD     D
            SHLD    ABIN
    ;
            ELSE                    ;AB OUT OF PHASE WITH SUM
            LDED    ABOUT
            DAD     D
            SHLD    ABOUT
    ;
            ENDIF
    ;
            LHLD    ABIN
            LDED    ABTICNT
            DAD     D
            SHLD    ABIN
    ;
            LHLD    ABOUT
            LDED    ABTOCNT
            DAD     D
        ·   SHLD    ABOUT
    ;
            LXI     H, 0
            SHLD    —ETIONT
            SHLD    —ETCCNT
    ;
    ;SUM: DO NOT SEPARATE INPHASE AND OUT OF PHASE VALUES
    ;       ADD THEM ALL
    ;
            LHLD    ABSSMD
            LDED    SMIO
            DAD     D
            SHLD    SMIO
    ;
    ;
            LHLD    SMIO
            LDED    SMTCNT
            DAD     D
            SHLD    SMTCNT
    ;
    ;
            LXI     H, 0
            SHLD    SMTCNT
            RET
    ;
    ;
    UPDT2:
    ;
    ;1. STORE RC COUNTS
    ;2. RC COUNTS <== RCTCNT
    ;3. RCTCNT <== 0
    ;4. STORE AB COUNTS
    ;5. AB COUNTS <== ABTCNT
    ;6. ABTCNT <== 0
```

```
        LHLD        RCIN
        SHLD        RCINVOL
    ;
        LHLD        RCOUT
        SHLD        RCOUTVOL
    ;
        LHLD        RCTICNT
        SHLD        RCIN
    ;
        LHLD        RCTOCNT
        SHLD        RCOUT
    ;
        LXI         H, 0
        SHLD        RCTICNT
        SHLD        RCTOCNT
    ;
        LHLD        ABIN
        SHLD        ABINVOL
    ;
        LHLD        ABOUT
        SHLD        ABOUTVOL
    ;
        LHLD        ABTICNT
        SHLD        ABIN
    ;
        LHLD        ABTOCNT
        SHLD        ABOUT
    ;
        LXI         H, 0
        SHLD        ABTICNT
        SHLD        ABTOCNT
    ;
    ;SUM: DO NOT SEPARATE INPHASE AND OUT OF PHASE
        LHLD        SMIO
        SHLD        SMVOL
    ;
    ;
    ;
        LHLD        SMTCNT
        SHLD        SMIO
    ;
    ;
        LXI         H, 0
        SHLD        SMTCNT
      RET
    ;
    ;
    ;
    ;
    UPDT3:
    ;
    ;1. ADD ABS(RCDERV) TO RCTCNT
    ;2. ADD ABS(ABDERV) TO ABTCNT
```

EP 0 256 115 B1

```
        LDA             RCINPHS
        ID (.A, IS, NZERO)        ;THEN INPHASE
          LHLD          ABSRCD
          LDED          RCTICNT
        DAD             D
          SHLD          RCTICNT
        ELSE
          LHLD          ABSRCD
          LDED          RCTICNT
        DAD             D
          SHLD          RCTOCNT
        ENDIF
;
        LDA             ABINPHS
        IF (.A, IS, NZERO)
          LHLD          ABSABD
          LDED          ABTICNT
          DAD           D
          SHLD          ABTICNT
        ELSE
          LHLD          ABSABD
          LDED          ABTOCNT
          DAD           D
          SHLD          ABTOCNT
        ENDIF
;
;SUM: DO NOT SEPARATE INPHASE AND OUT OF PHASE
;
          LHLD          ABSSMO
          LDED          SMTCNT
          DAD           D
          SHLD          SMTCNT
;
        RET
;
;
;

;
;
;
GTSIGN:
        LHLD            RCDERV          ;CURRENT RC DERIV VALUE
        MOV             A, H
        ANI             80H
        STA             RCSIGN  -
;
        LHLD            ABDERV          ;CURRENT AB DERIV VALUE
        MOV             A, H
        ANI             80H
        STA             ABSIGN
;
        LHLD            SMDERV
```

85

```
        MOV             A, H
        ANI             80H
        STA             SMSIGN
    ;
    ;
        MOV             B, A
        LDA             RCSIGN
        IF (.A, EQ, B)      ;THEN RC IMPHASE W/ SUM
          XRA           A
          CMA
          STA           RCINPHS
        ELSE
          XRA           A
          STA           RCINPHS
        ENDIF
    ;
        LDA             SMSIGN
        MOV             B, A
        LDA             ABSIGN
        IF (.A, EQ, .B)     ;THEN AB INPHASE W/ SUM
          XRA           A
          CMA
          STA           ABINPHS
        ELSE
          XRA           A
          STA           ABINPHS
        ENDIF
        RET
    ;
    ;
    ;
    ;
    ;
    ;
    ;
    ;
    ;
    ;
    DERIV5:
    ;*********************
    ;DERIV USING 9 POINTS
    ;*********************
    ;
    ;DERIVATIVE USING 5 POINTS
    ;
    ;SRCOFST HOLDS OFFSET TO GET Y5 FROM RAW DATA BUFFERS
    ;
    ;ASSUME: RCY1, RCY2, RCY3, RCY4, ABY1, ABY2, ABY3, ABY4 ALREADY SET
    ;
        LXI             D, RCBUFF
        LHLD            SRCOFST
        DAD             D
        MOV             E, M
        INX             H
```

86

```
        MOV         D, M
        SDED        RCY9
;
        LHLD        SRCOFST
        LXI         D, ABBUFF
        DAD         D
        MOV         E, M
        INX         H
        MOV         D, M
        SDED        ABY9
;
        LXI         B,RSIZE         ;UPDATE FOR NEXT TIME
        LHLD        SRCOFST
        CALL        PTRUPD
        SHLD        SRCOFST
;DERIV = (9—1)*4 + (8—2)*3 + (7—3)*2 + (6—4)
;
        LHLD        RCY9
        LDED        RCY1
        ORA         A
        DSBC        D
        DAD         H       ;(RCY9—RCY1) ← 2
        DAD         H       ;(RCY9—RCY1) ← 4
        PUSH        H
;
        LHLD        RCY8
        LDED        RCY2
        ORA         A
        DSBC        D
        MOV         E, L
        MOV         D, H
        DAD         H       ;*2
        DAD         D       ;*3
        POP         D
        DAD         D
        PUSH        H
;
        LHLD        RCY7
        LDED        RCY3
        ORA         A
        DSBC        D
        DAD         H
        POP         D
        DAD         D
        PUSH        H
;
        LHLD        RCY6
        LDED        RCY4
        ORA         A
        DSBC        D
        POP         D
        DAD         D
        SHLD        RCDERV
```

```
;
;*****: DIVIDE RCDERV BY 4 TO AVOID OVERFLOW WHEN INTEGRATING IT
    LHLD        RCDERV
    SRAR        H
    RARR        L
    SRAR        H
    RARR        L
    SHLD        RCDERV

;***
;DERIV = (9—1)*4 + (8—2)*3 + (7—8)*2 + (6—4)
;
    LHLD        ABY9
    LDED        ABY1
    ORA         A
    DSBC        D
    DAD         H           ;(ABY9—ABY1)*2
    DAD         H           ;(ABY9—ABY1)*4
    PUSH        H
;
    LHLD        ABY8
    LDED        ABY2
    ORA         A
    DSBC        D
    MOV         E, L
    MOV         D, H
    DAD         H           ;*2
    DAD         D           ;*3
    POP         D
    DAD         D
    PUSH        H
;
    LHLD        ABY7
    LDED        ABY3
    ORA         A
    DSBC        D
    DAD         H
    POP         D
    DAD         D
    PUSH        H
;
    LHLD        ABY6
    LDED        ABY4
    ORA         A
    DSBC        D
    POP         D
    DAD         D
    SHLD        ABDERV
;
;*****: DIVIDE ABDERV BY 4 TO AVOID OVERFLOW WHEN INTEGRATING IT
    LHLD        ABDERV
    SRAR        H
    RARR        L
```

88

```
        SRAR            H
        RARR            L
        SHLD            ABDERV

;***
;
;RESET VALUES FOR NEXT TIME
        LHLD            RCY2
        SHLD            RCY1
        LHLD            RCY3
        SHLD            RCY2
        LHLD            RCY4
        SHLD            RCY3
        LHLD            RCY5
        SHLD            RCY4
        LHLD            RCY6
        SHLD            RCY5
        LHLD            RCY7
        SHLD            RCY6
        LHLD·           RCY8
        SHLD            RCY7
        LHLD            RCY9
        SHLD            RCY8
;
        LHLD            ABY2
        SHLD            ABY1
        LHLD            ABY3
        SHLD            ABY2
        LHLD            ABY4
        SHLD            ABY3
        LHLD            ABY5
        SHLD            ABY4
        LHLD            ABY6
        SHLD            ABY5
        LHLD            ABY7
        SHLD            ABY6
        LHLD            ABY8
        SHLD            ABY7
        LHLD            ABY9
        SHLD            ABY8
;
;
        LHLD            RCDERV
        LDED            ABDERV
        DAD             D
        SHLD            SMDERV
;
;
;
;COMPUTE ABSOLUTE VALUE OF RCDERV AND ABDERV, AND SMDERV
        LHLD            RCDERV
        MOV             A, H
        ANI             80H
        IF(.A, IS, NZERO)       ;THEN RCDERV < 0
```

```
        MOV         A, L
        CMA
        MOV         L, A
        MOV         A, H
        CMA
        MOV         H, A
        INX         H
        SHLD        ABSRCD
    ELSE
        SHLD        ABSRCD
    ENDIF
;
    LHLD        ABDERV
    MOV         A, H
    ANI         80H
    IF (.A, IS, NZERO)      ;THEN ABDERV < 0
        MOV         A, L
        CMA
        MOV         L, A
        MOV         A, H
        CMA
        MOV         H, A
        INX         H
        SHLD        ABSABD
    ELSE
        SHLD        ABSABD
    ENDIF
;
    LHLD        SMDERV
    MOV         A, H
    ANI         80H
    IF (.A, IS, NZERO)      ;THEN SMDERV < 0
        MOV         A, L
        CMA
        MOV         L, A
        MOV         A, H
        CMA
        MOV         H, A
        INX         H
        SHLD        ABSSMD
    ELSE
        SHLD        ABSSMD
    ENDIF
;
;
;
    RET
;
;
;
;
;
;
```

90

```
;
DKGETP2:
;
;SEE IF AT LEAST 10 POINTS IN RING BUFFER
;
      LHLD          RIPT
      LXI           D, 20
      ORA           A
      DSBC          D
      IF (PSW, IS, CARRY)      ;THEN < 10 PTS
        STC
      ELSE
;
;
;
;SET UP INIT VALUES IN RCY1, RCY2, RCY3, RCY4
;     ABY1, ABY2, ABY3, ABY4, AND SRCOFST
;
      LXI           H, RCBUFF
      MOV           E, M
      INX           H
      MOV           D, M
      SDED          RCY1
;
      INX           H
      MOV           E, M
      INX           H
      MOV           D, M
      SDED          RCY2
;
      INX           H
      MOV           E, M
      INX           H
      MOV           D, M
      SDED          RCY3
;
      INX           H
      MOV           E, M
      INX           H
      MOV           D, M
      SDED          RCY4
;
      INX           H
      MOV           E, M
      INX           H
      MOV           D, M
      SDED          RCY5
;
      INX           H
      MOV           E, M
      INX           H
      MOV           D, M
      SDED          RCY6
;
```

```
        INX         H
        MOV         E, M
        INX         H
        MOV         D, M
        SDED        RCY7
    ;
        INX         H
        MOV         E, M
        INX         H
        MOV         D, M
        SDED        RCY3
    ;
    ;
        LXI         H, ABBUFF
        MOV         E, M
        INX         H
        MOV         D, M
        SDED        ABY1
    ;
        INX         H
        MOV         E, M
        INX         H
        MOV         D, M
        SDED        ABY2
    ;
        INX         H
        MOV         E, M
        INX         H
        MOV         D, M
        SDED        ABY3
    ;
        INX         H
        MOV         E, M
        INX         H
        MOV         D, M
        SDED        ABY4
    ;
        INX         H
        MOV         E, M
        INX         H
        MOV         D, M
        SDED        ABY5
    ;
        INX         H
        MOV         E, M
        INX         H
        MOV         D, M
        SDED        ABY6
    ;
        INX         H
        MOV         E, M
        INX         H
        MOV         D, M
```

```
    SDED        ABY7
;
    INX         H
    MOV         E, M
    INX         H
    MOV         D, M
    SDED        ABY8
;
    LXI         H, 14       ;OFSET FOR THE 9TH VALUE IN BUFFERS
    SHLD        SRCOFST
;
    LXI         H, B        ;OFFSET OF MIDDLE 1F
    SHLD        ROPT
;
    LXI         H, 0
    SHLD        INTGRC
     ORA        A
    ENDIF
    RET
;
    .END
```

```
        .IDENT          PRNTVAL
        .INSERT         FPMAC.SRC
        .INSERT         SP80.ASM
        .INSERT         CALCOM
        .INSERT         LP2COM
        .INSERT         SYCOM
        ;
        .EXTERN         PRTIME
        ;
        .EXTERN         LPON, LPOFF
        FINIT
        .EXTERN         ROUND
        .ENTRY          PRNTVAL
        ;
;THIS ROUTINE PRINTS OUT THE DELTA VALUES:
        ;
        ;     SUM       SP        SUM/SP
        ;
        ;
        ;
;THERE ARE (2*MAXONT) - 1 VALUES
        ;
        ;
        ;
PRNTVAL:
        ;
;ASSUME VALUE OF FONT IS IN AREG ON ENTRY TO THIS ROUTINE
        ;
        FSTA            FONT
;ONLY MAKE HARD COPY
GO1:
        LHLD            MAXONT
        DCX             H
        SLAR            L
        RALR            H
        SHLD            ONT
        LIQI            4
        LKI             H, XVALUES
        SHLD            X2PTR
        LXI             H, 0
        SHLD            CPPTR        ;OFFSET FOR SP, RO, AB
        XRA             A
        STA             PTF       ;THIS IS ZERO WHEN
                                  ;ODD # TIME THRU LOOP
        ;
        XRA             A
        CMA
        STA             HRDFLG
        ;
        MVI             A, 10
        STA             LONT
;ONLY MAKE HARD COPY
        CALL            LPON
```

```
;OUTPUT SEVERAL LINE FEEDS
     LXI          H, CRLFS
     CALL         TXTYP
     CALL         PRTIME
     FPRN         HEAD, HDPT
     CALL         LPOFF
;
LOOP1:
;
;GET THE DELTA FOR SPIROMETER
;
     LHLD         CPPTR
     LXI          D, SPMAX
     DAD          D
     MOV          E, M
     INX          H
     MOV          D, M
     SDED         SPVALUE
;
     LHLD ·       CPPTR
     LDA          PTF
     IF (.A, IS, NZERO)       ;THEN EVEN # THRU LOOP
                  INX     H
                  INX     H
     ENDIF
     LXI          D, SPMIN
     DAD          D
     MOV          E, M
     INX          H
     MOV          D, M
                              ;DE = MIN SP PT
     LHLD         SPVALUE ;HL = MAX SP PT
     ORA          A
     DSBC         D
     SHLD         SPDELT        ;SP DELTA
     FILA         SPDELT
     FLOT
     FLDB         CCS
     FMUL
     FSTA         SPDELT        ;NOW INTERMS OF ML
;
;NOW GET SUM DELTA VALUE
;
;
     LHLD         CPPTR
     LXI          D, ABMAX
     DAD          D
     MOV          E, M
     INX          H
     MOV          D, M
     SDED         ABVALUE
;
     LHLD         CPPTR
```

EP 0 256 115 B1

FILE: PRNTVAL.ASM

```
        LDA             PTF
        IF (.A, IS, NZERO)      ;THEN EVEN # THRU LOOP
                        INX     H
                        INX     H
        ENDIF
        LXI             D, ABMIN
        DAD             D
        MOV             E, M
        INX             H
        MOV             D, M
                                        ;DE = MIN SUM PT
        LHLD            ABVALUE         ;HL = MAX SUM PT
        ORA             A
        DSBC            D
        SHLD            ABDELT          ;SUM DELTA VALUE
        FILA            ABDELT
        FLOT
        FLOB            CCS
        FMUL
        FSTA            ABDELT          ;NOW IN ML
;
;
;NOW GET XVALUE (SUM/SP)
        LDQR            X2PTR
        FQWL
        FSTA            XVAL
        SDQR            X2PTR
;
;ONLY MAKE HARD COPY
        CALL            LPON
        LDA             PTF
        IF (.A, IS, ZERO)       ;THEN INSP
            LXI         H, ILAB
        ELSE
            LXI         H, ELAB
        ENDIF
        CALL            TXTYP
        FPRN            DELT, DELTPT
        CALL            LPOFF
;
;
;
        LDA             PTF
        IF (.A, IS, NZERO)      ;THEN EVEN # THRU LOOP
            LHLD        CPPTR
            INX         H
            INX         H
            SHLD        CPPTR
        ENDIF
        LDA             PTF
        CMA
        STA             PTF
```

96

```
        DSKZ            CNT, LOOP1
;
;
LP11:
;
;CALCULATE AND PRINT OUT THE MEAN, STD, ST ERROR, AND X ERROR
;
        FLDB            XSUM
        FLDA            FCNT
        FDIV
        FSTA            MEAN
;
;
        FLDB            XSUM
        FLDA            XSUM
        FMUL
        FATB
        FLDA            FCNT
        FDIV
        FLDB            XXSUM
        FSUB
        FATB
        FLDA            FONT
        FDIV
        FSQR
        FSTA            STDV
        FLDA            FCNT
        FSQR
        FLDB            STDV
        FDIV
        FSTA            STERR
;
        FLDB            MEAN
        FFOA            1
        FSUB
        FATB
        FFOA            100
        FMUL
        FSTA            PCERR
        FLDA            PCERR
        FABS
        FATB
        FFOA            1
        FSUB
        FSTA            FTEMP
        FTST            FTEMP, LT
        IF (PSW, IS, ZERO)      ;THEN ABS(ERROR) < 1
           FFOA         0
           FSTA         PCERR
        ENDIF
;
GO2:
;
```

97

```
;ONLY MAKE HARD COPY
        CALL            LPON
        FPRN            STATS, STPT
;OUTPUT SEVERAL LINE FEEDS
        LXI             H, CRLFS
        CALL            TXTYP
        CALL            LPOFF
;
;
    RET
;
;
STATS: .ASCIS   '
Mean SUM/SP        = ∂D3
Stdv SUM/SP        = ∂D3
St Err SUM/SP      = ∂D3

% ERROR            = ∂D1%

'
;
STPT: .WORD MEAN, STDV, STERR, PCERR
;
ILAB:   .ASCIS  '(I)
ELAB:   .ASCIS  '(E)
;
;
DELT:   .ASCIS  '∂D3    ∂D3    ∂D3
'
;
DELTPT:         .WORD    ABDELT, SPDELT, (VAL)
;
HDPT:
;
HEAD: .ASCIS '
        Validation:
        SUM     SP      SUM/SP
'
CRLFS: .ASCIS '
'
;
;
;
;
        .EXTERN PRT
        .LOC    PRT
FTEMP: .BLKB    4
;
SPDELT:         .BLKB 4
RCDELT:         .BLKB 4
Y2PTR: .BLKB    2
X2PTR: .BLKB    2
ABDELT:         .BLKB 4


LCNT:   .BLKB   1
FCNT:   .BLKB   4
;
MEAN: .BLKB 4
STDV:  .BLKB 4
STERR: .BLKB 4
PCERR: .BLKB 4
LOOPCNT:        .BLKB 1
;
        .RELOC
        .END
```

```
;
;IF VALIDATING AND IF USING SPIROBAG
;THEN SPVALUE HOLDS THE SUM MIN
                LDA         BAG
                IF (.A, IS, NZERO)        ;THEN USING SPIROBAG
                LDED        SPVALUE      ;SUM VALUE
                JMP         L2001
                ENDIF
;
                LDED        ABVALUE
L2001:
                MOV         M, E
                INX         H
                MOV         M, D
        ENDIF
    LDA                     PTF
    CMA
    STA                     PTF
    ENDIF
;
SKIP5:
;
EXIT:
;
    CALL                    CSTS
    IF (PSW, IS, NZERO)
        CALL                CINP
        IF (.A, EQ, CR)        ;IF CARRIAGE RETURN
            JMP             CLKSTP
        ENDIF
    ENDIF
;
    XRA                     A
    STA                     INTFLG
;
;********************
    POP                     PSW
    POP                     H
    POP                     D
    POP                     B
;
    RET
;
;
;
CLKTIK:
;
    LHLD                    ELPTM
    INX                     H
    SHLD                    ELPTM
    LDED                    SFREQ
    CALL                    TDHE
    IF (PSW, IS, ZERO)          ;THEN 1 SECON
```

```
        LXI             H, 0
        SHLD            ELPTM
        LHLD            SECONDS
        INX             H
        SHLD            SECONDS
        XRA             A
        CMA
        STA             SECFLG
        LXI             D, 60
        CALL            TDHE
        IF (PSW, IS, ZERO)      ;THEN 1 MINUTE
          LXI           H, 0
          SHLD          SECONDS
          LHLD          ELPMIN
          INX           H
          SHLD          ELPMIN
        ENDIF
     ENDIF
     RET
;
;
;
;
SAMPLE:
;
;
;
     LDA             BAG
     IF (.A, IS, ZERO)      ;THEN USING SPIROMETER
        LHLD          SMAD        ;SUM VALUE
        MOV           A, H
        CMA
        ANI           0FH
        MOV           H, A
        MOV           A, L
        CMA
        MOV           L, A
        SHLD          DATA
        LHLD          RIPT
        LXI           D, ABBUFF
        CALL          PRNG
     ;
     ;
        LHLD          SPAD        ;SPIROMETER VALUE
        MOV           A, H
        CMA
        ANI           0FH
        MOV           H, A
        MOV           A, L
        CMA
        MOV           L, A
        SHLD          DATA
        LDA           PNFLAG
        IF (.A, IS, NZERO)        ;THEN INPUTTING PNEUMOTACH-SO INTEGRATE
```

```
;NOTE:**** ASSUME 0 FLOW IS AT 7FFH
        LHLD          DATA
        LXI D, 7FFH
        ORA A
        DSBC          D
;DIVIDE BY 8 (SIGNED SHIFT RIGHT)
;CHANGE TO SHIFT ONLY 2 TIMES
;2BYTES PER INSTRUCTION
        NOP
        NOP
;
        NOP
        NOP
;SRAR H
;RARR L
        SRAR          H
        RARR          L
        SRAR          H
        RARR          L
        LDED          PNSUM
        DAD           D
        SHLD          PNSUM
;ADD OFFST SO ALL # > 0
        LXI   D, 800H
        DAD           D
        SHLD          DATA
        ENDIF
        CALL          CKFLIP
        LHLD          RIPT
        LXI           D, SPBUFF
        CALL          PRNG
;
        ELSE          ;USING FIXED VOLUME
;STORE SUM DATA IN SPBUFF SO WILL BE USED TO PICK OFF BREATHS
;
        LHLD          SMAD          ;SUM VALUE
        MOV           A, H
        CMA
        ANI           0FH
        MOV           H, A
        MOV           A, L
        CMA
        MOV           L, A
        SHLD          DATA
        LHLD          RIPT
        LXI           D, SPBUFF
        CALL          PRNG
;
;FILL ABBUFF WITH ZEROES
        LXI           H, 0
        SHLD          DATA
        LHLD          RIPT
        LXI           D, ABBUFF
```

```
          CALL          PRNG
      ;
      ;
          ENDIF
      ;
      ;
      ;FILL RCBUFF WITH ZEROES
          LXI           H, 0
          SHLD          DATA
          LHLD          RIPT
          LXI           D, RCBUFF
          CALL          PRNG
      ;
      ;
      ;
      ;
      ;
      ;INCREMENT RIPT FOR NEXT TIME
      ;
          LHLD     .    TIME
          INX           H
          SHLD          TIME
          SHLD          DATA
          LHLD          RIPT
          LXI           D, TMBUFF
          CALL          PRNG
      ;
          LHLD          RIPT
          SHLD          ODRIPT
          LXI           B, RSIZE
          LHLD          RIPT
          CALL          PTRUPD
          SHLD          RIPT
      ;
          LDA           WRAPF
          IF (.A, IS, ZERO)        ;SEE IF RIPT RESET TO 0
              LXI           D, 0
              LHLD          RIPT
              CALL          TDHE
          IF (PSW, IS, ZERO)
              MVI           A, 0FFH
              STA           WRAPF
          ENDIF

      ENDIF
      RET
      ;
      ;
      ;
CKFLIP:
      ;
      ;CHECK IF NEED TO FLIP
          LDA           FLIPV
          IF (.A, IS, NZERO)
```

102

In practice, the Respigraph™ scaling amplifiers for the rib cage and abdomen signals are initially set at unity gain. A 10 minute baseline is then taken. The program automatically computes the delta values for the rib cage and abdomen signals, discards the wild points, computes the mean values for the rib cage and abdomen from the totals of the remaining delta values, computes the standard deviations for those means, and then computes the proportionality factor Z from Equation M. The scaling amplifier for the rib cage is then adjusted to the proportionality factor Z. The real time output from the Respigraph™ representing the sum of the rib cage and abdomen signals is then proportional to tidal volume as explained more fully above. The program also recomputes the proportionality factor Z during eac subsequent 5 minute interval. If the recomputed value differs from 1.0 by more than an acceptable amount, the calibration routine can be rerun. The program also computes the scaling factor M from Equation N if instructed to do so upon inputting an actual tidal volume value as derived, e.g., by spirometry.

**Claims**

1. A method for calibrating an apparatus for non-invasively measuring a subject's respiration volume of the type including means for providing a signal rsponsive to a rib cage dimension indicative of rib cage contribution to respiration volume, meanks for providing a signal responsive to an abdominal dimension indicative of abdominal contribution to respiration volume, and means for multiplying at least one of said rib cage and abdominal signals by a predetermined weighting factor for reflecting the relative contributions of said rib cage and abdomen to respiration volume, whereby an information representative of the respiration volume can be obtained from the sum of the weighted rib cage and abdominal signals, the method being chartacterized in that said weighting factors are determined by:

(a) totaling delta values for said rib cage signal and said abdominal signal over a baseline period of substantially steady state breathing, said delta values being parameters of said rib cage and abdominal signals indicative of the relative amplitude of those signals for each of a plurality of breaths over said baseline period, and

(b) dividing an average variability of the mean of the total of said delta values for one of said rib cage or abdominal signals by an average variability of the mean of the total of said delta values for the other of said rib cage or abdominal signals,

and in that said other signal is multiplied by a weighting factor equaling the quotient derived from step (b).

2. A method according to claim 1, characterized in that it comprises, before said totaling steps, the steps of determining the average variability of the sum of the rib cage and corresponding abdominal signals during said baseline; and discarding the delta values for the rib cage and corresponding abdominal signals whose sum differs from said average variability of the sum by a predetermined amount.

3. A method according to any one of claims 1 and 2 characterized in that it comprises the step of summing said one signal and said weighted other signal to provide a sum signal proportional to respiration volume.

4. A method according to any one of claims 1 to 3, characterized in that it comprises the steps of repeating steps (a)—(b) at predetermined intervals using said weighted other signal; and providing an indication if said quotient differs from 1.0 by a predetermined amount.

5. A method according to claim 3, characterized in that it comprises the steps of providing a signal indicative of actual respiration volume over a finite time interval; dividing said actual respiration volume by said sum signal as derived during said time interval for obtaining a scaling factor; and thereafter multiplying said sum signal by said scaling factor for obtaining a signal quantitatively related to actual respiration volume.

6. An apparatus for calibrating a non-invasive apparatus for measuring a subject's respiration volume, said measuring apparatus being of the type including means for providing a signal responsive to a rib cage dimension indicative of rib cage contributon to respiration volume, means for providing a signal responsive to an abdominal dimension indicative of abdominal contribution to respiration volume, and means for multiplying at least one of said rib cage and abdominal signals by a predetermined weighting factor for reflecting the relative contributions of said rib cage and abdomen to respiration volume, whereby an information representative of the respiration volume can be obtained from the sum of the weighted rib cage and abdominal signals, said calibrating apparatus being characterized in that it comprises:

means for separating totaling delta values for said rib cage and abdominal signals over a baseline period of substantially steady state breathing, said delta values being parameters of said rib cage and abdominal signals indicative of the relative amplitude of those signals for each of a plurality of breaths over said baseline period,

means for dividing an average variability of the mean of the total of said delta values for one of said rib cage or abdominal signals by an average variability of the mean of the total of said delta values for the other signal,

means for adjusting said multiplying means for multiplying said other signal by the quotient determined by said dividing means.

7. An apparatus according to claim 6, characterized in that it further comprises means for determining the average variability of the sum of the rib cage and corresponding abdominal signals during the baseline period, and means for discarding the delta values for the rib cage and corresponding abdominal signals whose sum differs from said average variability of the sum by a predetermined amount.

8. An apparatus according to any one of claims 6 and 7, characterized in that it comprises means for summing said one signal and said weighted other signal for providing a sum signal proportional to respiration volume.

9. An apparatus according to any one of claims 6 to 8, characterized in that it comprises means for recalculating said quotient at predetermined intervals using said weighted other signal, and means for providing an indication if said quotient differs from 1.0 by a predetermined amount.

10. An apparatus according to claim 8, characterized in that it comprises: means for providing a signal indicative of actual respiration volume over a finite time interval; means for dividing said actual respiration volume signal by said sum signal as derived during said time interval for obtaining a scaling factor; and means for multiplying said sum signal by said scaling factor for obtaining a signal quantitatively related to actual respiration volume.

**Patentansprüche**

1. Verfahren zum Kalibrieren einer Vorrichtung für nichteindringende Messungen eines Atmungsvolumens eines Subjektes, von der Art mit einer Einrichtung zum Liefern eines Signals, welches auf eine Rippenkorbdimension anspricht und den Rippenkorbbeitrag zum Atmungsvolumen anzeigt, einer Einrichtung zur Lieferung eines Signals, welches auf eine Abdominaldimension anspricht und den Abdominalbeitrag zum Atmungsvolumen anzeigt, und Einrichtung zum Multiplizieren von wenigstens einem der genannten Rippenkorb- und Abdominal signale mit einem vorbestimmten Wichtungsfaktor, um die relativen Anteile des genannten Rippenkorbs und des Abdomens zu dem Atmungsvolumen zu reflektieren, wodurch eine Information aus der Summe der gewichteten Rippenkorb- und Abdominalsignale erhalten werden kann, die repräsentativ für das Atmungsvolumen ist, welches Verfahren dadurch gekennzeichnet ist, daß die Wichtungsfaktoren bestimmt werden durch

(a) Summenbildung der Deltawerte des genannten Rippenkorbsignals und des genannten Abdominalsignals über einer Zeitbasisperiode eines im wesentlichen gleichmäßigen Atmungszustandes, welche genannten Deltawerte Parameter der genannten Rippenkorb- und Abdominalsignale sind, welche die relative Amplitude jener Signale für jede der Vielzahl von Atmungen über der genannten Zeitbasisperiode anzeigen, und

(b) Teilen einer durchschnittlichen Variabilität des Mittelwertes der Summe der genannten Deltawerte für entweder die Rippenkorb- oder die Abdominalsignale durch eine durchschnittliche Variabilität des Mittelwertes der Summe der genannten Deltawerte von dem anderen der genannten Rippenkorb- oder Abdominalsignale,

und daß das genannte andere Signal mit einem Wichtsungsfaktor multipliziert wird, welcher gleich dem Quotienten ist, der von dem Schritt (b) abgeleitet ist.

2. Verfahren nach Anspruch 1, dadurch gekennzeichnet, daß es, vor den Summenbildungsschritten, die Schritte der Bestimmung der durchschnittlichen Variabilität der Summe der Rippenkorb- und entsprechenden Abdominalsignale während der genannten Zeitbasis umfaßt; und Ausscheiden der Deltawerte für die Rippenkorb- und die entsprechenden Abdominalsignale, deren Summe von der genannten durchschnittlichen Variabilität der Summe um einen vorbestimmten Betrag verschieden ist.

3. Verfahren nach einem der Ansprüche 1 und 2, dadurch gekennzeichnet, daß es den Schritt der Summenbildung des genannten einen Signale und der genannten gewichteten anderen Signals umfaßt, um ein Summensignal zu liefern, das proportional zum Atmungsvolumen ist.

4. Verfahren nach einem der Ansprüche 1 bis 3, dadurch gekennzeichnet, daß es die Schritte der Wiederholung der Schritte (a)—(b) zu vorbestimmten Intervallen umfaßt, unter Verwendung des genannten gewichteten anderen Signals; und die Lieferung einer Anzeige, falls der genannte Quotient um einen vorbestimmten Betrag von 1,0 verschieden ist.

5. Verfahren nach Anspruch 3, dadurch gekennzeichnet, daß es umfaßt: die Schritte des Lieferns eines Signals, das tatsächliche Atmungsvolumen über ein endliches Zeitintervall anzeigt; die Teilung des genannten tatsächlichen Atmungsvolumens durch das genannte Summensignal, wie es während des genannten Zeitintervalls abgeleitet wird, um einen Normierungsfaktor zu erhalten; und danach die Multiplikation des genannten Summensignals mit dem genannten Normierungsfaktor, um ein Signal zu erhalten, welches in quantitativer Relation zu dem tatsächlichen Atmungsvolumen steht.

6. Vorrichtung zum Kalibrieren einer nicht eindringenden Vorrichtung zum Messen des Atmungsvolumens eines Subjektes, welche genannte Meßvorrichtung von dem Typ ist, der eine Einrichtung zum Liefern eines Signales umfaßt, das auf die Rippenkorbdimension anspricht und den Rippenkorbbeitrag zum Atmungsvolumen anzeigt, und eine Einrichtung zum Liefern eines Signals, welches auf eine Abdominaldimension anspricht und den Abdominalbeitrag zum Atmungsvolumen anzeigt, und Einrichtungen zum Multiplizieren von wenigstens einem der Rippenkorb- und Abdominalsignale mit einem vorbestimmten Wichtungsfaktor, um die relativen Beiträge von Rippenkorb und Abdomen zum Atmungsvolumen zu reflektieren, wodurch eine Information aus der Summe der gewichteten Rippenkorb- und Abdominalsignale erhalten werden kann, welche für das Atmungsvolumen repräsentativ ist, welche genannte Kalibriervorrichtung dadurch gekennzeichnet ist, daß sie umfaßt:

Einrichtungen, um separat die Summen der Deltawerte für die genannten Rippenkorbsignale und die Abdominalsignale über einer Zeitbasisperiode eines im wesentlichen gleichmäßigen Atmungszustandes zu bilden, welche genannten Deltawerte Parameter der genannten Rippenkorb- und Abdominalsignale sind, die die relative Amplitude jener Signale für jede der Vielzahl von Atmungen während der genannten Zeitbasisperiode anzeigen,

Einrichtungen zum Teilen der durchschnittlichen Variabilität des Mittelwertes der Summe der genannten Deltawerte für eines der Rippenkorb- oder Abdominalsignale durch eine durchschnittliche Variabilität des Mittelwertes der Summe der genannten Deltawerte für das andere Signal, und

Einrichtungen zur Justierung der genannten Multiplikationseinrichtung zum Multiplizieren des genannten anderen Signals mit dem Quotienten, der durch die genannte Teilungseinrichtung bestimmt ist.

7. Vorrichtung nach Anspruch 6, dadurch gekennzeichnet, daß sie ferner Einrichtungen zum Bestimmen der durchschnittlichen Variabilität der Summe der Rippenkorb- und der entsprechenden Abdominalsignale während der Zeitbasisperiode umfaßt, und Einrichtungen zum Ausscheiden der Deltawerte der Rippenkorb- und der entsprechenden Abdominalsignale, deren Summme von der genannten durchschnittlichen Variabilität der Summe um einen vorbestimmten Betrag unterschiedlich ist.

8. Vorrichtung nach einem der Ansprüche 6 und 7, dadurch gekennzeichnet, daß sie Einrichtungen zur Summenbildung des genannten einen Signals und des genannten gewichteten anderen Signals umfaßt, um ein Summensignal zu liefern, das proportional zum Atmungsvolumen ist.

9. Vorrichtung nach einem der Ansprüche 6 bis 8, dadurch gekennzeichnet, daß sie Einrichtungen zur Nachberechnung des genannten Quotienten zu vorbestimmten Intervallen unter Verwendung des genannten gewichteten anderen Signals umfaßt, und Einrichtungen zum Liefern einer Anzeige, falls der genannte Quotient um einen vorbestimmten Betrag von 1,0 verschieden ist.

10. Vorrichtung nach Anspruch 8, dadurch gekennzeichnet, daß sie umfaßt: Einrichtungen zum Liefern eines Signals, das das tatsächliche Atmungsvolumen über ein endliches Zeitinvervall anzeigt; Einrichtungen zum Teilen des genannten tatsächlichen Atmungsvolumensignals durch das genannte Summensignal, wie es während des genannten Zeitintervalls abgeleitet wird, um einen Normierungsfaktor zu erhalten, und Einrichtungen zum Multiplizieren des genannten Summensignals durch den genannten Normierungsfaktor, um ein Signal zu erhalten, welches in quantitativer Relation zu dem tatsächlichen Atmungsvolumen steht.

**Revendications**

1. Procédé permettant d'étalonner un appareil de mesure non invasion du volume respiratoire d'un sujet, du type comportant un moyen permettant de produire un signal qui répond à une dimension de la cage thoracique indicative de la contribution de la cage thoracique au volume respiratoire, um moyen permettant de produire un signal qui répond à une dimension de l'abdomen indicative de la contribution abdominale au volume respiratoire, et un moyen servant à multiplier au moins un desdits signaux de la cage thoracique et de l'abdomen par un facteur de pondération prédéterminé afin de réfléchir les contributions relatives de la cage thoracique et de l'abdomen au volume respiratoire, de sorte qu'une information représentative du volume respiratoire peut être obtenue à partir de la somme des signaux pondérés de la cage thoracique et de l'abdomen, le procédé étant caractérisé en ce que lesdits facteurs de pondération sont déterminés par:

(a) la totalisation de valeurs delta relatives audit signal de la cage thoracique et audit signal abdominal sur une durée de ligne de base d'une respiration effectuée dans un état sensiblement stationnaire, lesdits valeurs delta étant des paramètres desdits signaux de la cage thoracique et de l'abdomen qui sont indicatifs de l'amplitude relative de ces signaux pour chacune de plusieurs respirations effectuées sur ladite durée de ligne de base, et

(b) la division de la dispersion moyenne de la moyenne du total desdites valeurs delta pour un premier desdits signaux de la cage thoracique et de l'abdomen par la dispersion moyenne de la moyenne du total desdites valeurs delta pour l'autre desdits signaux de la cage thoracique et de l'abdomen,

et en ce que ledit autre signal est multiplié par un facteur de pondération égal au quotient obtenu à l'étape (b).

2. Procédé selon la revendication 1, caractérisé en ce qu'il comprend, avant lesdites étapes de totalisation, les étapes consistant à déterminer la dispersion moyenne de la somme des signaux de la cage thoracique et des signaux correspondants de l'abdomen pendant ladite ligne de base; et à écarter les valeurs delta de signaux de la cage thoracique et des signaux correspondants de l'abdomen dont la somme diffère, par rapport à ladite dispersion moyenne de la somme, d'une quantité prédéterminée.

3. Procédé selon l'une quelconque des revendications 1 et 2, caractérisé en ce qu'il comprend l'étape consistant à additionner ledit premier signal et ledit autre signal pondéré afin de produire un signal de somme proportionnel au volume respiratoire.

4. Procédé selon l'une quelconque des revendications 1 à 3, caractérisé en ce qu'il comprend les étapes consistant à répéter les étapes (a)—(b) à intervalles prédéterminés à l'aide dudit autre signal pondéré; et à fournir une indication si ledit quotient diffère, par rapport à la valeur 1,0, d'une quantité prédéterminée.

5. Procédé selon la revendication 3, caractérisé en ce qu'il comprend les étapes consistant à fournir un signal indicatif du volume respiratoire réel sur un intervalle de temps fini; à diviser ledit volume respiratoire réel par ledit signal de somme tel qu'il est obtenu pendant ledit intervalle de temps afin de calculer un facteur de démultiplication; et, ensuite, à multiplier ledit signal de somme par ledit facteur de démultiplication afin d'obtenir un signal quantitativement relié au volume respiratoire réel.

6. Appareil permettant d'étalonner un appareil non invasif de mesure du volume respiratoire d'un sujet, ledit appareil de mesure étant du type qui comporte un moyen permettant de produire un signal qui répond à une dimension de la cage thoracique indicative de la contribution de la cage thoracique au volume respiratoire, un moyen permettant de produire un signal qui répond à une dimension de l'abdomen indicative de la contribution de l'abdomen au volume respiratoire, et un moyen servant à multiplier au moins l'un desdits signaux de la cage thoracique et de l'abdomen par un facteur de pondération prédéterminé afin de réflécher les contributions relatives de la cage thoracique et de l'abdomène au volume respiratoire, de sorte qu'une information représentative du volume respiratoire peut être obtenue à partir de la somme des signaux pondérés de la cage thoracique et de l'abdomen, ledit appareil d'étalonnage étant caractérisé en ce qu'il comprend:

un moyen servant à totaliser séparément des valeurs delta relatives auxdits signaux de la cage thoracique et de l'abdomen sur une durée de ligne de base d'une respiration effectuée dans un état sensiblement stationnaire, lesdites valeurs delta étant des paramètre desdits signaux de la cage thoracique et de l'abdomen qui sont indicatifs de l'amplitude de ces signaux pour chacune de plusieurs respirations effectuées sur ladite durée de la ligne de base,

un moyen servant à diviser la dispersion moyenne de la moyenne du total desdites valeurs delta pour un premier. desdits signaux de la cage thoracique et de l'abdomen par la dispersion moyenne de la moyenne du total desdites valeurs delta pour l'autre signal, et

un moyen servant à adjuster ledit moyen de multiplication afin qu'il multiplie ledit autre signal par le quotient déterminé par ledit moyen de division.

7. Appareil selon la revendication 6, caractérisé en ce qu'il comprend en outre un moyen permettant de déterminer la dispersion moyenne de la somme des signaux de la cage thoracique et de signaux abdominaux corespondants pendant la durée de la ligne de base, et un moyen permettant d'écarter les valeurs delta des signaux de la cage thoracique et des signaux abdominaux correspondants dont la somme différe, par rapport à ladite dispersion moyenne de la somme, d'une quantité prédéterminée.

8. Appareil selon l'une quelconque des revendications 6 et 7, caractérisé en ce qu'il comprend un moyen servant à additionner ledit premier signal et ledit autre signal pondéré afin de produire un signal de somme proporitonnel au volume respiratoire.

9. Appareil selon l'une quelconque des revendications 6 et 8, caractérisé en ce qu'il comprend un moyen permettant de recalculer ledit quotient à intervalles prédéterminés à l'aide dudit autre signal pondéré, et un moyen servant à fournir une indication si ledit quotient différe, par rapport à la valeur 1,0, d'une quantité prédéterminée.

10. Appareil selon la revendication 8, caractérisé en ce qu'il comprend: un moyen servant à produire un signal indicatif du volume respiratoire réel sur un intervalle de temps fini; un moyen servant à divise le signal du volume respiratoire réel par ledit signal de somme tel qu'il a été obtenu pendant ledit intervalle de temps afin de produire un facteur de démultiplication; et un moyen servant à multiplier ledit signal de somme par ledit facteur de démultiplication afin de produire un signal quantitativement relié au volume respiratoire réel.

# FIG. 1

FIG. 2

FIG. 3

RC

AB

$\Delta_1$  $\Delta_2$

$\Delta_1$  $\Delta_2$

t

EP 0 256 115 B1